# EUROPEAN PATENT APPLICATION

(11) **EP 2 045 005 A2**
(43) Date of publication of application: **08.04.2009**
(21) Application number: 08168964.8
(22) Date of filing: 03.07.2001
(51) Int. Cl.: B01J 19/00, B01L 3/00

(54) **Devices and methods for carrying out chemical reactions using photogenerated reagents**

(30) Priority: 03.07.2000 US 215719 P
(62) Divisional of application: 01952397.6
(71) Applicant: Xeotron Corporation, Houston, TX 77054 (US)
(72) Inventor: Zhou, Xiaochuan, Houston, TX 77030-4322 (US); Zhou, Tiecheng, Pearland, TX 77584 (US); Sun, David, Bellaire, TX 77401 (US)
(74) Representative: Harrison Goddard Foote

(57) **Abstract**

Fluidic methods and devices for conducting parallel chemical reactions are disclosed. The methods are based on the use of in situ photogenerated reagents such as photogenerated acids, photogenerated bases, or any other suitable chemical compounds that produce active reagents upon light radiation. The present invention describes devices and methods for performing a large number of parallel chemical reactions without the use of a large number of valves, pumps and other complicated fluidic components. The present invention provides microfluidic devices that contain a plurality of microscopic vessels for carrying out discrete chemical reactions. Other applications may include the preparation of microarrays of DNA and RNA oligonucleotides, peptides, oligosacchrides, phospholipids and other biopolymers on a substrate surface for assessing gene sequence information, screening for biological and chemical activities, identifying intermolecular complex formation, and determining structural features of molecular complexes.

## Description

### FIELD OF THE INVENTION

The present invention relates to the field of chemical fluidic reactors for parallel performance of pluralities of chemical reactions and parallel synthesis of pluralities of chemical compounds. More particularly, this invention relates to devices and methods for distributing liquids, implementing discrete photochemical reactions for in situ production of reagents, and activating discrete chemical and biochemical reactions.

### BACKGROUND OF THE INVENTION

Modern drug development, disease diagnosis, gene discovery, and various genetic-related technologies and research increasingly rely on making, screening, and assaying a large number of chemical and/or biochemical compounds. Traditional methods of making and examining the compounds one at a time are becoming increasingly inadequate. Therefore there is a need for chemical/biochemical reaction systems to perform high-throughput synthesis and assay, chemical and biochemcal reactions including DNA hybridization and hydrogen-bonding reactions.

Parallel synthesis and analysis of chemical/biochemical compounds in a microarray form is one of the most efficient and effective high-throughput methods. Light-directed on-chip parallel synthesis combining semiconductor-based photolithography technologies with solid-phase organic chemistry has been developed for making very-large-scale microarrays of oligonucleotides and peptides (Pirrung et al., U.S. Patent No. 5,143,854). The microarrays have provided libraries of synthetic molecules for screening biological activities (Pease et al., Proc. Natl. Acad. Sci. USA 91, 5022-5026 (1994)).

Pirrung et al. describe a method of oligonucleotide synthesis on a planar substrate coated with linker molecules. The linker molecule terminus contains a reactive functional group such as hydroxyl group protected with a photoremovable-protective group. Using a photomask-based lithographic method, the photoremovable-protecting group is exposed to light through the first photomask and removed from the linker molecules in selected regions. The substrate is washed and then contacted with a phosphoramidite monomer that reacts with exposed hydroxyl groups on the linker molecules. Each phosphoramidite monomer molecule contains a photoremovable-protective group at its hydroxyl terminus. Using the second photomask, the substrate is then exposed to light and the process repeated until an oligonucleotide array is formed such that all desired oligonucleotide molecules are formed at predetermined sites. The oligonucleotide array can then be tested for biologic activity by being exposed to a biological receptor having a fluorescent tag, and the whole array is incubated with the receptor. If the receptor binds to any oligonucleotide molecule in the array, the site of the fluorescent tag can be detected optically. This fluorescence data can be transmitted to a computer, which computes which oligonucleotide molecules reacted and the degree of reaction.

The above method has several significant drawbacks for the synthesis of molecular arrays: (a) synthesis chemistry involving the use of photoremovable-protective groups is complicated and expensive; (b) synthesis has lower stepwise yields (the yield for each monomer addition step) than conventional method and is incapable of producing high purity oligomer products; (c) a large number of photomasks are required for the photolithography process (up to 80 photomasks for making a microarray containing oligonucleotides of 20 bases long) and therefore, the method is expensive and inflexible for changing microarray designs.

Another approach for conducting parallel chemical/biochemical reactions relies on the use of microfluidic devices containing valves, pumps, constrictors, mixers and other structures (Zanzucchi et al. U.S. Patent No. 5,846,396). These fluidic devices control the delivery of chemical reagents of different amounts and/or different kinds into individual corresponding reaction vessels so as to facilitate different chemical reactions in the individual reaction vessels. The method allows the use of conventional chemistry and therefore, is capable of handling varieties of chemical/biochemical reactions. However, this type of fluidic device is complicated and its manufacturing cost is high. Therefore, the method is not suitable for making low-cost chemical/biochemical microarrays.

The present invention simplifies the structure of fluidic devices for parallel performance of discrete chemical reactions by using a newly developed chemical approach for conducting light-directed chemical reactions (Gao et al., J. Am. Chem. Soc. 120, 12698-12699 (1998) and WO09941007A2). It was discovered that by replacing a standard acid (such as trichloroacetic acid) with an in-situ photogenerated acid (PGA) in the deblock reaction of an otherwise conventional DNA synthesis one can effectively use light to control the synthesis of DNA oligomer molecules on a solid support. The photoacid precursor and the produced acid were both in solution phase. The main advantages of the new method include the minimum change to the well-established conventional synthesis procedure, commercial availability and low cost for the chemical reagents involved, and high yield comparable to that achievable with conventional synthesis procedure. This method can be extended to control or initiate other chemical/biochemical reactions by light with the use of various properly chosen photogenerated reagents (PGR), such as photogenerated acids and bases.

Methods of parallel synthesis of microarrays of various molecules on a solid surface using PGR were previously disclosed by Gao et al. in WO09941007A2, the teaching of which is incorporated herein by reference. An important step in the parallel synthesis is the formation of discrete reaction sites on the solid surface such that the reagents generated by photolytic processes would be confined in the selected sites during the time the photogenerated reagents participate in chemical reactions. Physical barriers and patterned low surface-tension films were used to form isolated microwells and droplets, respectively on the solid surface. The methods are effective for preventing crosstalk (mass transfer due to an diffusion and/or fluid flow) between adjacent reaction sites. However, during the time the photogenerated reagents are generated and participate in the corresponding reactions the liquid confined at the reaction sites has to remain essentially static, meaning no fluid flow during the reactions. This lack of fluid flow could limit the mass transfer between the reactive reagents in the liquid and the reactive solid surface and therefore, could adversely affect the corresponding reaction rate.

Another potential problem with the above method is the possible side-reactions due to the production of free radicals during light exposures. In addition, the reactive solid surface is often a part of a transparent window through which light radiation is applied and therefore, undesirable photon-induced degradation of the synthesized molecules on the solid surface could take place.

Therefore, improvements are desired in the following areas: enhancing mass transfer while keeping discrete reaction sites isolated, reducing the possibility of radical-induced side reactions, and avoiding radiation-induced degradation reactions. Preferably, these are all achieved at once with the use of simple and low-cost fluidic device structures.

### SUMMARY OF THE INVENTION

In one aspect, an improved microfluidic reactor is provided comprising a plurality of flow-through reaction cells for parallel chemical reactions, each reaction cell comprising (i) at least one illumination chamber, and (ii) at least one reaction chamber, wherein the illumination chamber and the reaction chamber are in flow communication and are spatially separated in the reaction cell.

In another aspect, an improved microfluidic reactor is provided comprising a plurality of flow-through photoillumination reaction cells for parallel chemical reactions in fluid communication with at least one inlet channel and at least one outlet channel.

In still other aspects, additional microfluidic reactor embodiments are provided, as well as methods of using and methods of preparing the improved microfluidic reactors.

### BRIEF DESCRIPTION OF THE DRAWINGS

FIG. 1A schematically illustrates the operation principle of a flowthrough reactor system using photogenerated reagents. Illumination and photogenerated-reagent-involved chemical/biochemical reactions are carried out in a reaction cell having separated illumination and reaction chambers.
FIG. 1B schematically illustrates the operation principle of a flowthrough reactor system for performing parallel chemical reactions using photogenerated reagents. Illumination and photogenerated-reagent-involved chemical/biochemical reactions are carried out in a reaction cell having separated illumination and reaction chambers.
FIG. 1C schematically illustrates the operation principle of a flowthrough reactor system using photogenerated reagents. Illumination and photogenerated-reagent-involved chemical/biochemical reactions are carried out in a reaction cell, wherein the illumination chamber and the reaction chamber are combined.
FIG. 1D schematically illustrates the operation principle of a flowthrough reactor system for performing parallel chemical reactions using photogenerated reagents.

Illumination and photogenerated-reagent-involved chemical/biochemical reactions are carried out in a reaction cell, wherein the illumination chamber and the reaction chamber are combined.
FIG. 2A schematically illustrates the flow-path of a two-level device configuration for a single-inlet-single-outlet flowthrough multi-cell reactor system.
FIG. 2B schematically illustrates the flow-path of a two-level device configuration for a single-inlet-multiple-outlet flowthrough multi-cell reactor system.
FIG. 2C schematically illustrates the flow-path of a one-level device configuration for single-inlet-single-outlet flowthrough multi-cell reactor system.
FIG. 2D schematically illustrates the flow-path of a one-level device configuration for single-inlet-multiple-outlet flowthrough multi-cell reactor system.
FIG. 3A is an exploded perspective view of a flowthrough multi-cell reactor device that embodies the present invention.
FIG. 3B schematically illustrates the cross-section of microfluidic device shown in FIG. 3A and the operation principle of the device.
FIG. 3C schematically illustrates a variation of a flowthrough multiple-cell reactor shown in FIG. 3B with immobilized chemical compounds attached to both the inner surface of a window and the top surface of reaction chambers. This variation also contains a shadow mask on the inner surface of the window.
FIG. 3D is an exploded perspective view of a flowthrough multiple-cell reactor device involving vertical capillary reaction chambers.
FIG. 4A is an exploded perspective view of a high-density flowthrough multi-cell reactor device that embodies the present invention.
FIG. 4B schematically illustrates the cross-section of microfluidic device shown in FIG. 4A and the operation principle of the device.
FIG. 5A is an exploded perspective view of a one-level flowthrough multi-cell reactor device that embodies the present invention.
FIG. 5B schematically illustrates the first cross-section of the microfluidic device shown in FIG. 5A and the operation principle of the device.
FIG. 5C schematically illustrates the second cross-section of the microfluidic device shown in FIG. 5A and the operation principle of the device.
FIG. 5D schematically illustrates the cross-section of the microfluidic device shown in FIG. 5A with the internal surfaces of the reaction chambers coated with thin layers of substrate materials.
FIG. 5E schematically illustrates the microfluidic array chip device comprising the microfluidic structure shown in FIG. 5A, binary fluidic distribution channels, and inlet and outlet ports.
FIG. 5F schematically illustrates the microfluidic array chip device containing two arrays for multiple-sample assay applications.
FIG. 5G schematically illustrates the microfluidic array chip device containing tapered fluid channels.
FIG. 5H schematically illustrates the microfluidic array chip device containing another variation of tapered fluid channels.
FIG. 6A is an exploded perspective view of a high-density, one-level flowthrough multi-cell reactor device that embodies the present invention.
FIG. 6B schematically illustrates the cross-section of the microfluidic device shown in FIG. 6A and the operation principle of the device.
FIG. 7A schematically illustrates a variation of a flowthrough multi-cell reactor with reaction chambers containing beads in which solid-phase chemical reactions take place.
FIG. 7B schematically illustrates a variation of a flowthrough multi-cell reactor with reaction chambers containing pads in which solid-phase chemical reactions take place.
FIG. 8 schematically illustrates the flow-path of a single-inlet-multiple-outlet flowthrough multi-cell reactor system with reaction chambers containing beads in which solid-phase chemical reactions take place.
FIG. 9A is an exploded perspective view of a microfluidic device filled with the first liquid (the liquid can be seen in FIG. 9D).
FIG. 9B schematically illustrates the perspective view of a microfluidic device when the second liquid is sent in through the first fluid channel while no flow is allowed in the second fluid channel (the liquid can be seen in FIG. 9E).
FIG. 9C schematically illustrates the perspective view of a microfluidic device when the second liquid is sent in through the second fluid channel while no flow is allowed in the first fluid channel (the liquid can be seen in FIG. 9F).
FIG. 9D schematically illustrates the cross-section of the microfluidic device shown in FIG. 9A. The device is filled with the first liquid.
FIG. 9E schematically illustrates the cross-section of the microfluidic device of FIG. 9B after the first set of fluid channels are filled with the second liquid.
FIG. 9F schematically illustrates the cross-section of the microfluidic device of FIG. 9C after the second set of channels are filled with the second liquid.
FIG. 9G schematically illustrates fluid structures that allow a fluid to pass through liquid channels.
FIG. 10A schematically shows an exploded perspective view of a microfluidic multi-cell reactor device that has been fabricated.
FIG. 10B shows a wafer substrate as the starting material for a microfluidic template.
FIG. 10C shows a slab substrate after the first etching step during the fabrication of a microfluidic template.
FIG. 10D shows a slab substrate after the second etching step during the fabrication of a microfluidic template.
FIG. 10E shows a completed microfluidic template.
FIG. 10F shows a photograph of a completed microfluidic array device.
FIG. 11 shows a fluorescence image of an oligonucleotide array.
FIG. 12 shows a fluorescence image of an oligonucleotide array hybridized with fluorescein labeled targets.

### DETAILED DESCRIPTION OF THE INVENTION

### Definition of Terms

The term "photogenerated-reagent precursor" (PRP) refers to a chemical compound that produces one or more reactive chemical reagents when it is irradiated or illuminated with photons of certain wavelengths. The wavelengths may be in any appropriate regions of infrared, visible, ultraviolet, or x-ray.

The term "photogenerated-acid precursor" (PGAP) refers to a chemical compound that produces acids when it is irradiated or illuminated with photons of certain wavelengths. The wavelengths may be in any appropriate regions of infrared, visible, ultraviolet, or x-ray.

The term "photogenerated-acid" (PGA) refers to an acid that is produced from PGAP under irradiations or illuminations with photons of certain wavelengths. The wavelengths may be in any appropriate regions of infrared, visible, ultraviolet, or x-ray.

The term "photogenerated reagent" (PGR) refers to a chemical compound that is produced from the irradiation or illumination of a photogenerated-reagent precursor. In most of the cases, PGR is a reactive reagent in the concerned chemical or biochemical reactions. However, the term may be used to refer to any chemical compounds that are derived from the irradiation of the photogenerated reagent precursor and may or may not be reactive in certain chemical/biochemical reactions.

The term "probe molecule" refers to a ligand molecule that is employed to bind to other chemical entities and form a larger chemical complex so that the existence of said chemical entities could be detected. Preferably, within a suitable window of chemical and physical conditions, such as pH, salt concentration, and temperature, the probe molecule selectively bind to other chemical entities of specific chemical sequences, specific conformations, and any other specific chemical or physical properties.

### Approaches

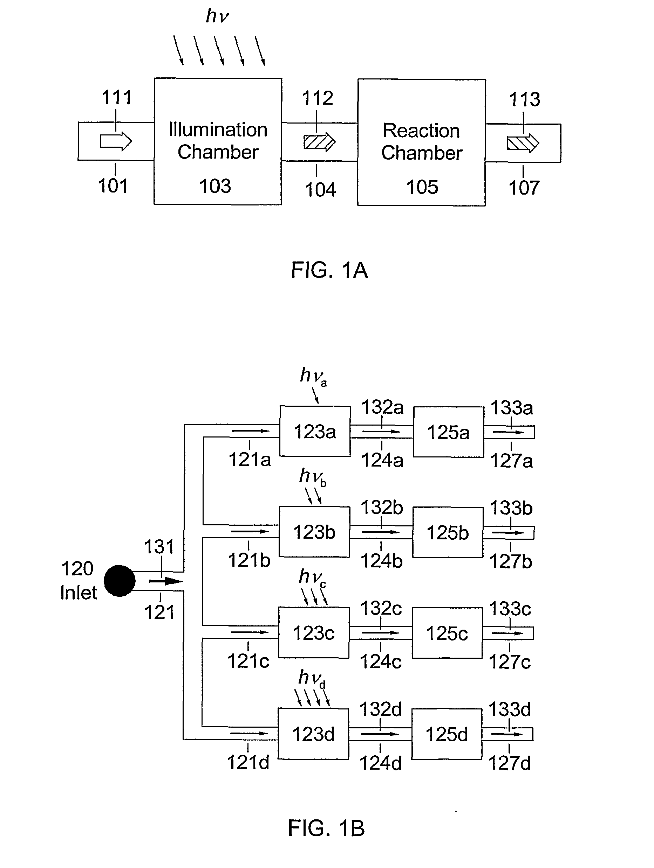

The present invention provides a method of performing parallel chemical/biochemical reactions in discrete reaction vessels. One preferred aspect of the present invention is the use of in situ generated chemical reagents to affect and/or cause interested chemical/biochemical reactions. FIG. 1A schematically illustrates the operation principle of a flowthrough reactor system using photogenerated reagents. A solution **111** containing at least one photogenerated reagent precursor flows through an inlet channel **101** into an illumination chamber **103.** A light exposure, hv, causes the generation of active chemical reagents from the photogenerated reagent precursor in the illumination chamber **103.** The active chemical reagent containing solution **112** then flows through a connection channel **104** into a reaction chamber **105,** which contains reactive compounds and/or substances either in a solution phase or on a solid phase substrate, to cause a chemical/biochemical reaction(s). The reactive compounds and/or substances in the reaction chamber **105** may be immobilized in the chamber or delivered into the chamber through a separate channel (not shown in FIG. 1A). After the chemical/biochemical reaction(s), an effluent **113** flows out the reactor system through an outlet channel **107.**

In one aspect of the present invention, the illumination chamber **103** and the reaction chamber **105,** which are part of a reaction cell, are spatially separated so that light exposure hv is prevented from being applied into the reaction chamber **105.** In addition, after coming out the illumination chamber **103,** preferably the solution **112** spends a sufficient amount time in the connection channel **104** so that any free radicals that may be generated in the illumination chamber **103** would be deactivated before the solution **112** entering the reaction chamber **105.** The preferred time for the solution **112** to spend in the connection channel **104** is longer than the half lifetime of the free radicals. The more preferred time for the solution **112** to spend in the connection channel **104** is longer than twice the half lifetime of the free radicals. This would minimize the possibility of undesirable free-radical-induced side-reactions from taking place in the reaction chamber **105** of the reaction cell.

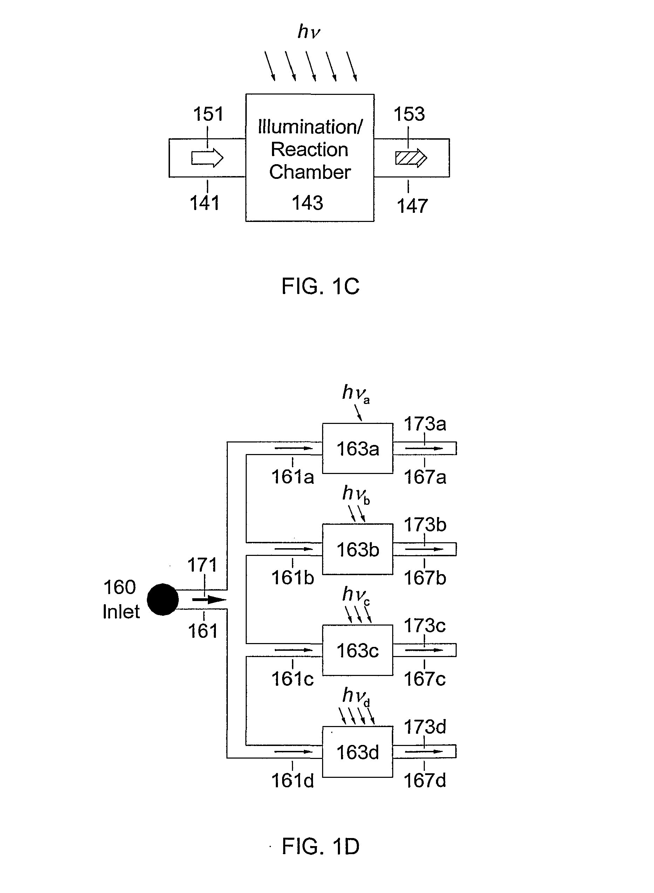

It should be understood that the present invention does not exclude the situation in which the illumination chamber **103** and the reaction chamber **105** of the reaction cell are partially or fully overlapping each. FIG. 1C illustrates schematically illustrates a reactor system having a reaction cell that accommodates light illumination and chemical/biochemical reaction in a one chamber **143.** Such an overlapping scheme is preferred in certain circumstances when, for example, the overlapping allows simpler and/or cheaper reactor devices to be fabricated. In embodiments having a full overlap, the term reaction cell and reaction chamber can be used interchangeably as there is a single combined chamber.

FIG. 1B schematically illustrates the operation principle of a flowthrough reactor system for performing parallel chemical reactions using photogenerated reagents. A solution **131** containing at least one photogenerated reagent precursor flows into the reactor system through an inlet **120.** The solution **131** then goes through a common inlet channel **121** and branch inlet channels **121a, 121b, 121c,** and **121d** and enters illumination chambers **123a, 123b, 123c,** and **123d,** respectively, of the reaction cell. Predetermined light exposures hνₐ, hν_{b}, hν_{c}, and hν_{d}, are applied to the corresponding illumination chambers **123a, 123b, 123c,** and **123d,** and cause the generation of active chemical reagents from the photogenerated reagent precursor. In one embodiment of the present invention, all light exposures contain the same wavelength distribution and are different only by their intensities. Under this scenario, preferably, the light exposures and the concentration of the photogenerated reagent precursor in the solution **131** are adjusted in such a way that the amounts of the produced active chemical reagents are proportional to the amounts or intensities of the light exposures. Thus, the produced solutions **132a, 132b, 132c,** and **132d** contain corresponding concentrations of active chemical-reagents. The solutions **132a, 132b, 132c,** and **132d** then flow through connection channels **124a, 124b, 124c,** and **124d** into corresponding reaction chambers **125a, 125b, 125c,** and **125d,** of the reaction cells which contains reactive compounds and/or substances either in a solution phase or on a solid phase substrate, to cause corresponding degrees of chemical/biochemical reactions. The reactive compounds and/or substances in the reaction chambers **125a, 125b, 125c,** and **125d** of the reaction cells may be immobilized in the chambers or delivered into the chambers through separate channels (not shown in FIG. 1B). Effluents **133a, 133b, 133c,** and **133d** then flow out the reactor system through outlet channels **127a, 127b, 127c,** and **127d.**

In another embodiment of the present invention, the solution **131** contains more than one photogenerated reagent precursors that have different excitation wavelengths and produce different chemical reagents. In this case, by using exposures hνₐ, hν_{b}, hν_{c}, and hν_{d} of different wavelength distributions different chemical reagents are produced in the corresponding illumination chambers **123a, 123b, 123c,** and **123d.** Thus, different types of chemical reactions can be carried out simultaneously in the corresponding reaction chambers **125a, 125b, 125c,** and **125d.** The present invention can be used to carry out as many parallel chemical reactions as one desires and as experimental conditions permit.

For certain applications, in which light exposure does not cause significant adverse chemical/biochemical reactions or simplified reactor structure is the primary consideration, it may not be necessary to have separate illumination and reaction chambers in the reaction cells. FIG. 1D schematically illustrates a reactor system for performing parallel chemical reactions that accommodates light illumination and chemical/biochemical reaction in single cells or chambers **163a, 163b, 163c,** and **163d.**

### Device Structures

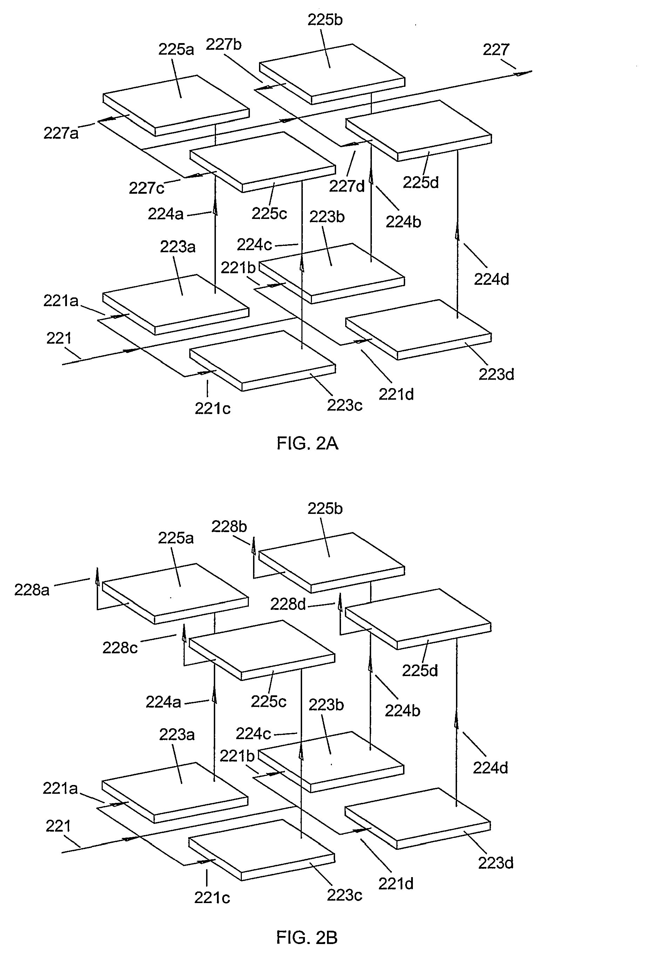

FIG. 2A schematically illustrates a two-level device configuration for a single-inlet-single-outlet flowthrough multi-cell reactor system. A common inlet **221,** branch inlets **221a, 221b, 221c,** and **221d,** and illumination chambers **223a, 223b, 223c,** and **223d** are placed at the first level. Connection channels **224a, 224b, 224c,** and **224d** connect the illumination chambers at the first level with reaction chambers **225a, 225b, 225c,** and **225d,** respectively, at the second level of the reaction cell. Effluents from the individual reaction chambers flow through outlets **227a, 227b, 227c,** and **227d,** merge into a common outlet **227,** and flow out the reactor system. With this configuration, each reaction cell, which consists of an illumination chamber, a connection channel, and a reaction chamber, provides a host for an individual chemical/biochemical reaction to take place. The configuration is particularly suitable for conducting parallel solid-phase chemical/biochemical reactions and/or synthesis in which reaction products remain on the solid supports/surfaces and effluents from individual reaction cells do not need to be individually collected. With only one common inlet and one common outlet, the reactor system is easy to construct and operate and is especially suitable for low cost applications.

A typical application for the reactor system shown in FIG. 2A usually involves many reaction and rinse steps in addition to the steps involving photochemical reactions. For most of the steps, especially for those involving photochemical reactions, the arrows in FIG. 2A point to the direction of the fluid flow. However, during some steps, especially for those requiring extended reagent contact or agitation, reverse flows are allowed or even desirable. In the design and construction of actual devices based on the configuration shown in FIG. 2A, measures should be taken to avoid crosstalk (chemical intermixing) between the reaction cells during photochemical reaction. For example, channels and inlets should be sufficiently long so that back-diffusion from the illumination chambers **223a, 223b, 223c,** and **223d** into the common inlet **221** is negligible. The determination of the suitable length of the inlets **221a, 221b, 221c,** and **221d** is based on the diffusion rate and fluid residence time in the inlets and is well-known to those skilled in the art of fluid flow and mass transfer.

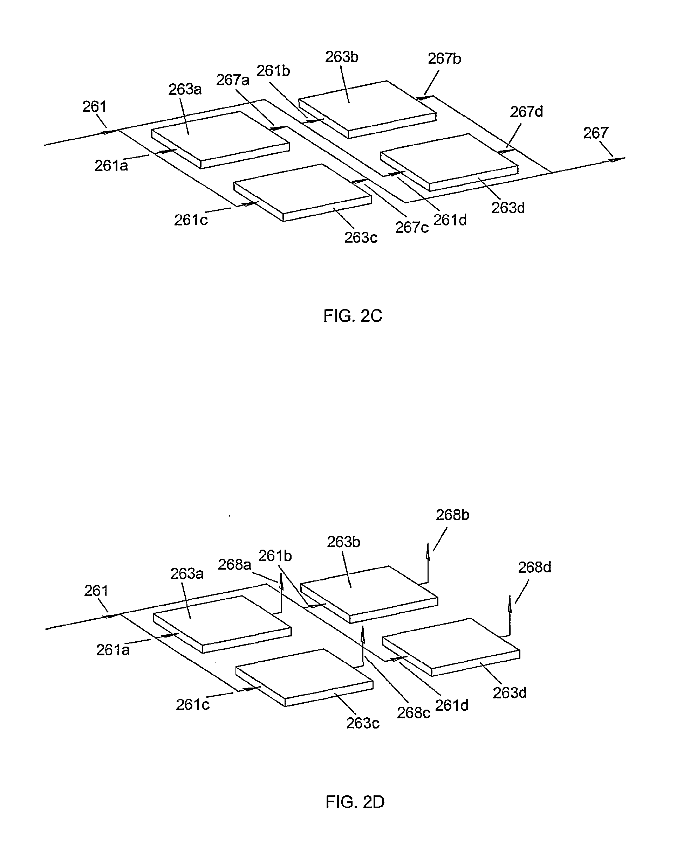

For certain applications, in which light exposure dose not cause significant adverse chemical/biochemical reactions or simplified reactor structure is the primary consideration, the construction of the reactor can be further simplified by combining corresponding illumination chambers with reaction chambers of the cells to form a one-level device configuration as shown in FIG. 2C. A fluid flows through a common inlet **261,** branch inlets **261a, 261b, 261c,** and **261d,** into individual reaction cells **263a, 263b, 263c,** and **263d,** which function as both illumination chambers and reaction chambers. Effluents from the individual reaction cells flow through outlets **267a, 267b, 267c,** and **267d,** merge into a common outlet **267,** and flow out the reactor system.

FIG. 2B schematically illustrates a two-level device configuration for a single-inlet-multiple-outlet flowthrough multi-cell reactor system. With this configuration, effluents from individual reactor chamber **225a, 225b, 225c,** and **225d** can be collected at corresponding outlets **228a, 228b, 228c,** and **228d** while the rest of the device structures and functions are similar to those shown in FIG. 2A. This configuration is particularly suitable for those applications in which chemical/biochemical reaction products are in solution phase and need to be individually collected for analysis or for other uses.

FIG. 2D schematically illustrates a one-level device configuration for a single-inlet-multiple-outlet flowthrough multi-cell reactor system. Most of the structures and functions of this configuration are similar to those shown in FIG. 2B with the exception that effluents from individual reaction cells **263a, 263b, 263c,** and **263d** are collected at corresponding outlets **268a, 268b, 268c,** and **268d.** This configuration is a preferred embodiment of the present invention for applications in which light exposure dose not cause significant adverse chemical/biochemical reactions or simplified reactor structure is the primary consideration.

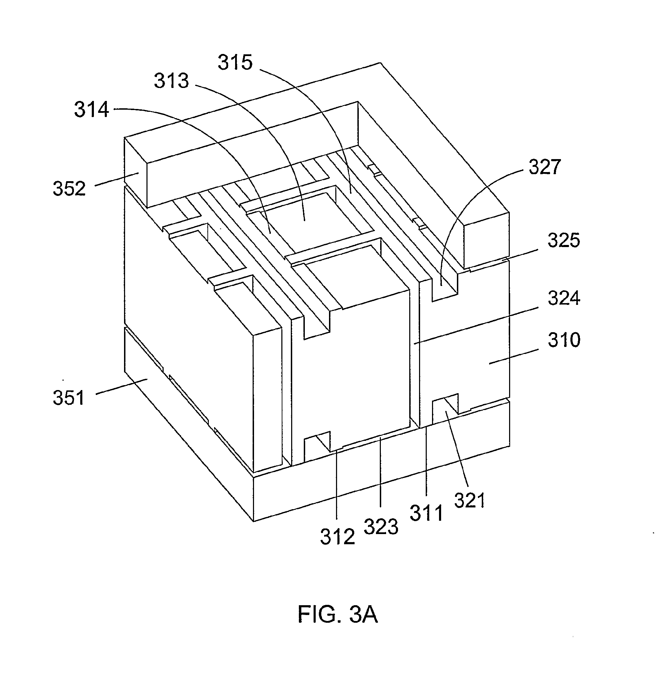

FIG. 3A illustrates an exploded perspective view of a flowthrough multi-cell reactor device, a preferred embodiment of the present invention. In this device, a microfluidic template **310** is sandwiched between a first window plate **351** and a second window plate **361.** Preferably, the microfluidic template **310** is made of silicon when reaction cells are small. In this case, the preferred distance between adjacent reaction cells is in the range of 10 to 5,000 µm. More preferably, the distance is in the range of 10 to 2,000 µm. Yet more preferably, the distance is in the range of 10 to 500 µm. Even more preferably, the distance is in the range of 10 to 200 µm. The silicon microfluidic template **310** is formed using etching processes which are well know to those skilled in the art of semiconductor processes and microfabrication (Madou, M., *Fundamentals of Microfabrication*, CRC Press, New York, (1997)). The top surface **313** of the microfluidic template **310** is preferably coated with silicon dioxide, which can be made by either oxidation or evaporation during a fabrication process. When the reaction cells are large, e.g. the distance between adjacent reaction cells is larger than 5,000 µm, plastic materials are preferred. Plastic materials may also be preferred for large quantity production of the multi-cell reactor device even when the distance between adjacent reaction cells is less than 5,000 µm. Preferred plastics include but are not limited to polyethylene, polypropylene, polyvinylidine fluoride, and polytetrafluoroethylene. The plastic microfluidic template **310** can be made using molding methods, which are well know to those skilled in the art of plastic processing. The one aspect of the present invention, the first window plate **351** and the second window plate **361** are preferably made of transparent glass and are bonded with the microfluidic template **310.** In another aspect of the present invention, the first window plate **351** and the second window plate **361** are preferably made of transparent plastics including but not limited to polystyrene, acrylic, and polycarbonate, which have the advantage of low cost and easy handing.

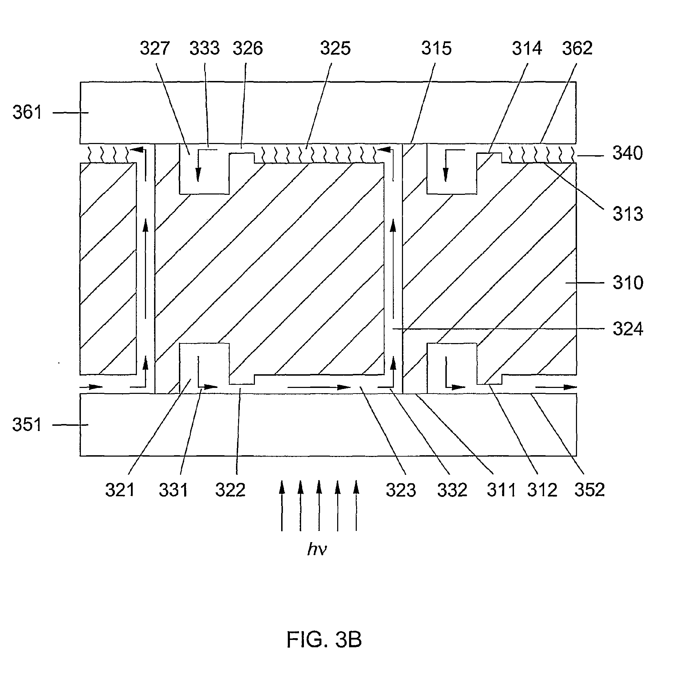

The microfluidic device shown in FIG. 3A embodies the two-level device configuration shown in FIG. 2A. The topographic structure of the bottom part of the microfluidic template 310, which cannot be seen in the figure, is a mirror image of the top part, which can be seen in the figure. FIG. 3B schematically illustrates the cross-section of the microfluidic device shown in FIG. 3A and the operation principle of the device. The first window plate **351** and the second window plate **361** are bonded or attached with the microfluidic template **310** at the bonding areas **311** and **315** of the microfluidic template **310.** Bonding or attaching can be done by covalent or non-covalent methods. During a reaction involving the use of photogenerated reagents, a feed solution **331** containing photogenerated reagent precursor flows from an inlet **321** through an inlet restriction gap **322** into an illumination chamber **323.** After an exposure hv in the illumination chamber **323,** active chemical reagents are produced and the resultant reactive solution **332** flows through a connection channel **324** into a reaction chamber **325.** In the reaction chamber **325** the reactive solution **332** is in contact with immobilized molecules **340** on the top surface **313** of the microfluidic template **310.** Chemical reactions take place between the active reagents in the reactive solution **332** and the immobilized molecules **340.** Then the solution flows through an outlet restriction gap **326** into the outlet **327** as an effluent **333.**

The function of the inlet restriction gap **322,** formed between a ridge **312** of the microfluidic template **310** and the inner surface **352** of the first window plate **351,** is to prevent any chemical reagents generated inside the illumination chamber **325** from going back into the inlet **321** region. Similarly, the outlet restriction gap **326,** which is formed between a ridge **314** on the microfluidic template 310 and inner surface **362** of the second window plate **361,** is to prevent any chemical reagents in the outlet **327** region from going into the reaction chamber **325.** This is achieved when the mass transfer rate due to fluid flow in the narrow restriction gaps is larger than that due to diffusion.

In a preferred embodiment, the cross-section areas of inlet **321** and outlet **327** channels are large enough so that the pressure drops along the channels are significantly lower than that across each individual reaction cell, which includes an inlet restriction gap **322,** an illumination chamber **323,** a connection channel **324,** a reaction chamber **325,** and an outlet restriction gap **326.** In addition, all reaction cells in each reactor system are preferably designed and constructed identically. These measures are necessary in order to achieve the same flow rates and therefore, the uniform reaction conditions in all reaction cells.

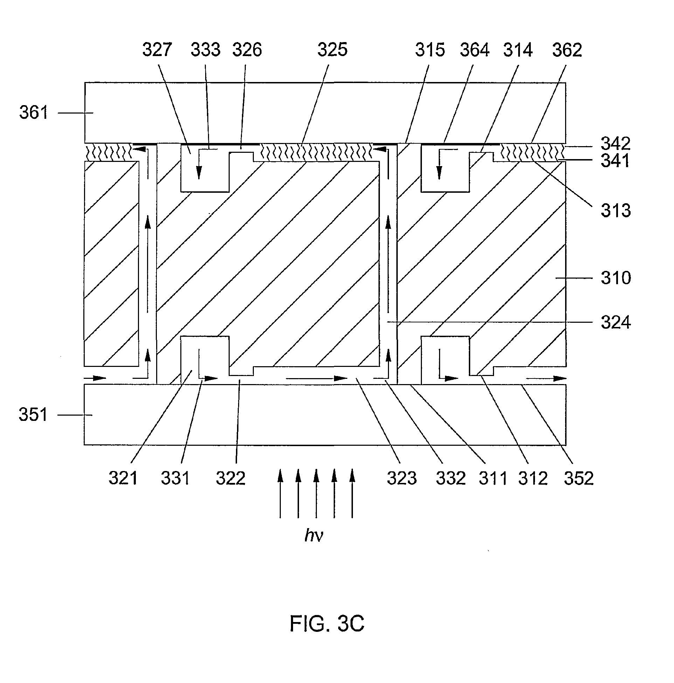

FIG. 3C schematically illustrates the cross-section of a modified microfluidic device. A shadow mask **364** is added to the inner surface **362** of the second window **361.** The shadow mask **364** eliminates potential optical interference from the topographic features of the microfluidic template **310** during an optical measurement, such as fluorescence imaging, which is performed through the second window **361.** The shadow mask **364** can be made of a thin film a metal or any other appropriate opaque materials, including but not limited to chromium, aluminum, titanium, and silicon. The film can be readily made by various well know thin film deposition methods such as electron-beam evaporation, sputtering, chemical vapor deposition, and vacuum vapor deposition, which are well know to those skilled in the art of semiconductor processes and microfabrication (Madou, M., Fundamentals of Microfabrication, CRC Press, New York, (1997)).

Another aspect of the present invention shown in FIG. 3C is the use of immobilized molecules **341** and **342** on both the top surface **313** of the microfluidic template **310** and the inner surface **362** of second window **361,** respectively. In assay applications of the microfluidic devices in which the immobilized molecules **341** and **342** are used as probe molecules, the use of double-layer configuration has the advantage of increasing area density of the probes and, therefore, increasing assay sensitivities.

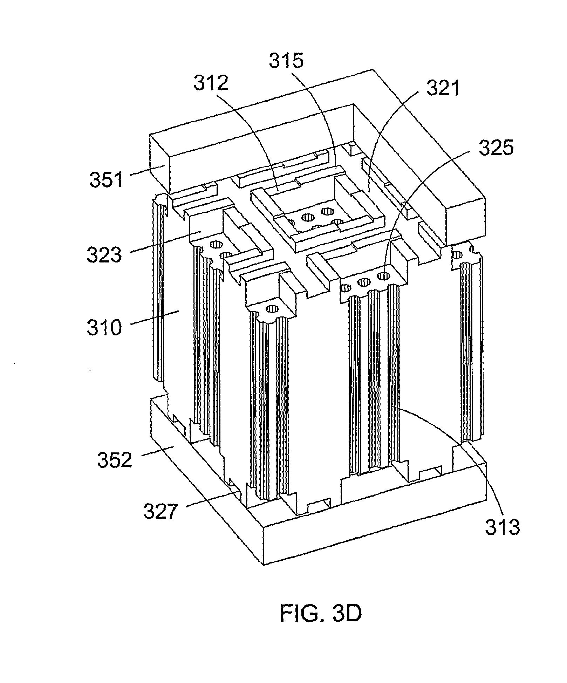

FIG. 3D shows another variation of the microfluidic device of the present invention. In this variation, reaction chambers **325** are in capillary form with the immobilized molecules (not shown in the figure) on the vertical walls **313** of the reaction chambers **325.** The preferred diameters of the capillaries are between 0.05 to 500 micrometers. More preferred diameters of the capillaries are between 0.1 to 100 micrometers. The main advantage of this variation is the possibility of having increased surface area of the reaction chamber walls **313,** as compared to the variation shown in FIG. 3A. For assay applications, the increased surface area facilitates the increased amount of immobilized molecules and therefore has the potential to increase the sensitivity of the assay. During a light directed chemical synthesis process, a reagent solution (not shown in FIG. 3D) flows through the inlet fluid channel **321** and into the illumination chamber **323.** When the reagent solution contains a photogenerated reagent precursor and when the predetermined illumination chambers **323** are exposed to light through a transparent window **351,** reactive reagents are generated and flow down into reaction chamber **325** where chemical reactions take place between the reactive reagents and immobilized molecules on the vertical walls **313.** The reagent fluid flows out through outlet fluid channels **327.**

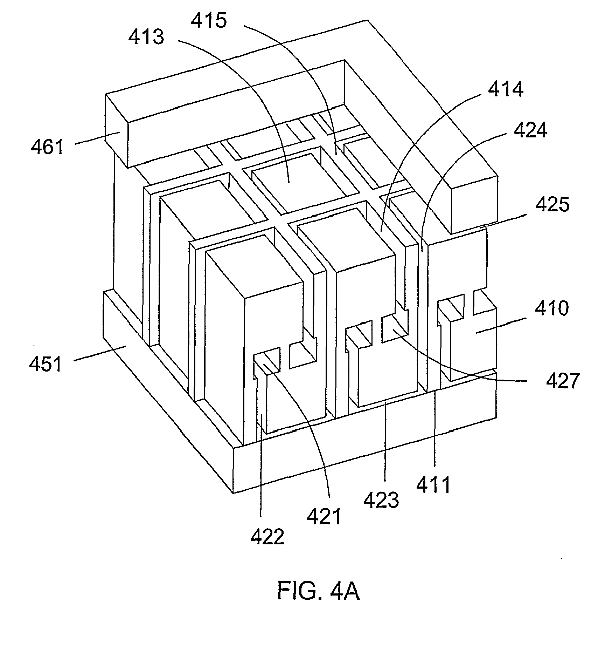

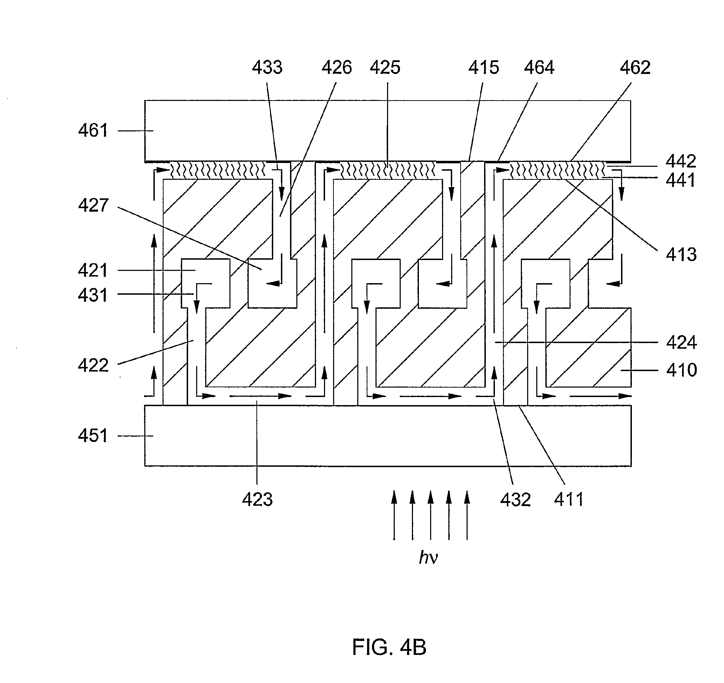

FIG. 4A illustrates an exploded perspective view of a high-density flowthrough multi-cell reactor device that embodies the two-level device configuration shown in FIG. 2A. FIG. 4B illustrates schematically the cross-section of the device shown in FIG. 4A. Compared to the device structure shown in FIG. 3A the device structure shown in FIG. 4A has a higher area density of the reaction chamber **425** and illumination chamber **423.** Inlet channel **421** and outlet channel **427** are embedded in the mid-section of the microfluidic template **410** so as to permit the upper and lower surface areas of the microfluidic template **410** fully utilized for implementing reaction and illumination chambers, respectively. During a reaction involving the use of photogenerated reagents, a feed solution **431** containing photogenerated reagent precursor flows from an inlet duct **422** into an illumination chamber **423.** After an exposure hv in the illumination chamber **423** through the first window plate **451,** active chemical reagents are produced and the resulted reactive solution **432** flows through a connection channel **424** into a reaction chamber **425.** In the reaction chamber **425** the reactive solution **432** is in contact with immobilized molecules **441** and **442** on the top surface **413** of the microfluidic template **410** and the inner surface **462** of the second window plate **461,** respectively. Chemical reactions take place between the active reagents in the reactive solution **432** and the immobilized molecules **441** and **442.** Then the effluent **433** flows through an outlet duct **426** into the outlet channel **427.**

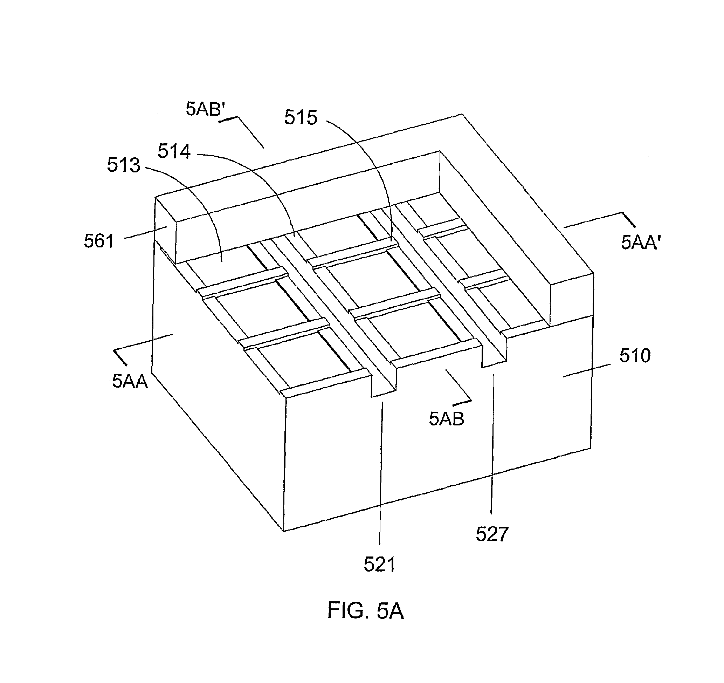

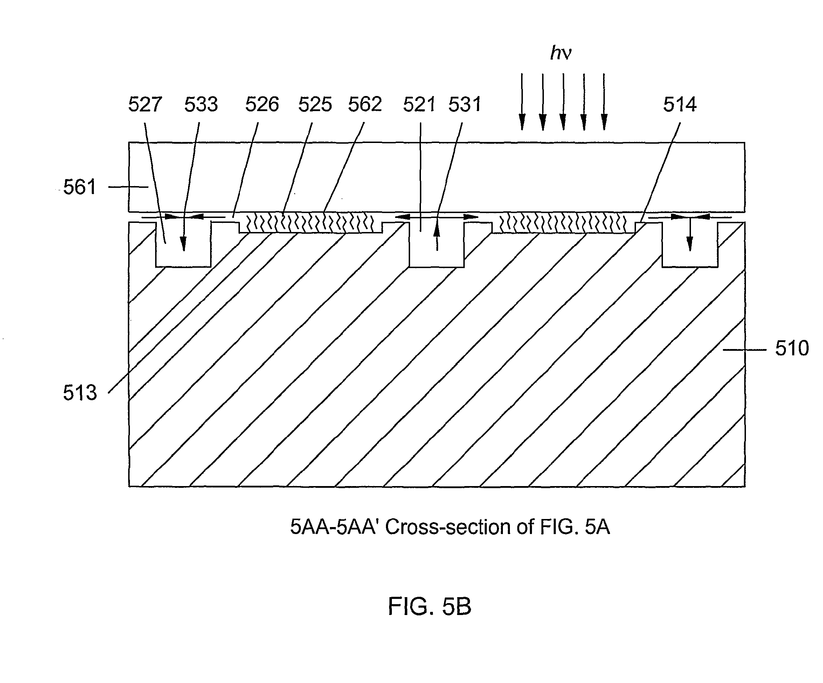

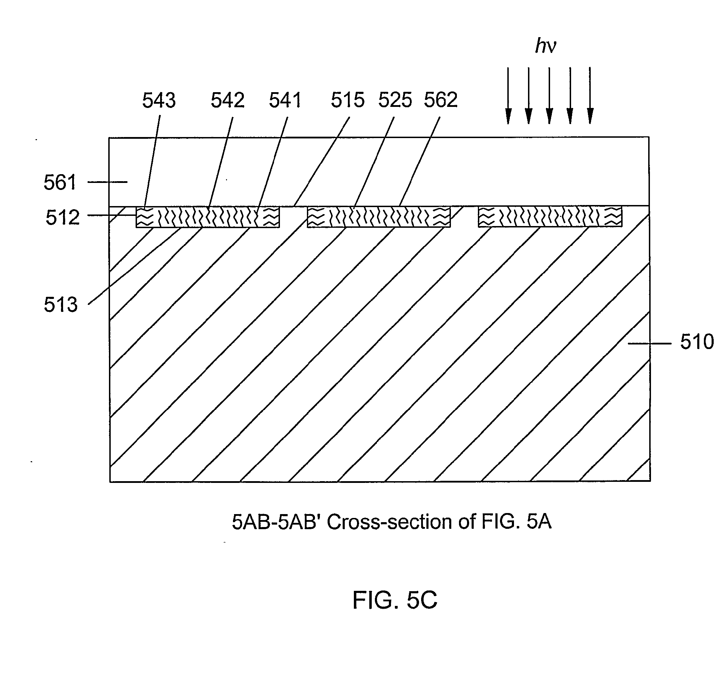

FIG. 5A illustrates an exploded perspective view of a flowthrough multi-cell reactor device, which embodies the one-level device configuration shown in FIG. 2C. FIG. 5B and FIG. 5C schematically illustrate the cross-section of the device shown in FIG. 5A. Microfluidic structures are formed between a microfluidic template **510** and a window plate **561,** bonded at the bonding area **515.** In this embodiment, light exposure and photogenerated-reagent-involved (PGRI) chemical/biochemical reaction are performed in a combined reaction chamber or cell **525.** Inlet channel **521** and outlet channel **527** are both located on one side of the microfluidic template **510.** The advantage of this device configuration is the simplification of the device structure and therefore the potential for a low manufacturing cost.

FIG. 5C schematically illustrates three-dimensional attachment of immobilized molecules **541, 542** and **543** on all four sides of the internal surface of a three-dimensional reaction chamber or cell **525.** The reaction chamber **525** is formed by the inner surface **562** of the window plate **561,** the upper surface of top surface **513** of the fluidic template **510,** and side walls **512.** For assay applications of the microfluidic devices the immobilized molecules **541, 542** and **543** are used as probe molecules. The three-dimensional attachment shown in FIG. 5C increases the amount of the probe molecules, as compared to that on a planar surface and therefore, increases assay sensitivity.

During a reaction involving the use of photogenerated reagents, a feed solution **531** containing photogenerated reagent precursor flows from an inlet channel **521** into a reaction chamber **525.** When the reaction chamber is illuminated, at least one active reagent is produced, which then react with immobilized molecules **541, 542,** and **542.** The effluent **533** flows through an outlet restriction gap **526** into the outlet channel **527.** The ridge **514** on the fluidic template **510** forms a flow restriction gap **526** in the inlet and outlet side of the reaction chamber **525.**

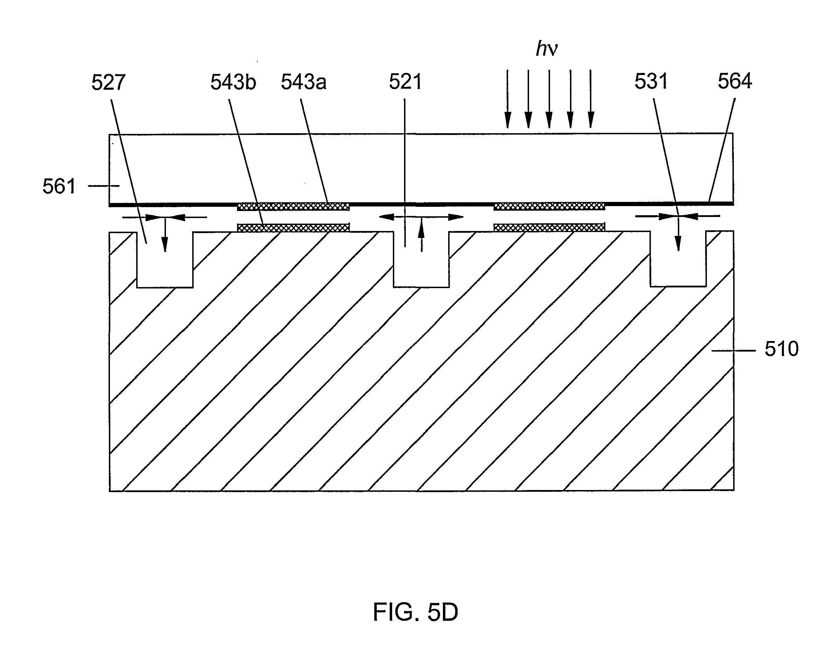

The microfluidic array devices of this invention can be used to produce or immobilize molecules at increased quantities by incorporating porous films 543a and 543b in the reaction chambers or cells as shown in FIG. 5D. Several materials and fabrication processes, which are well known to those skilled in the art of solid phase synthesis (A Practical Guide to Combinatorial Chemistry", edited by Czarnik et al., American Chemical Society, 1997, incorporated herein by reference), can be used to form the porous films inside the device. One process is to form a controlled porous glass film on the silicon wafer, which forms the fluidic template **510,** during the device fabrication process. In the first preferred process, a borosilicate glass film is deposited by plasma vapor deposition on the silicon wafer. The wafer is thermally annealed to form segregated regions of boron and silicon oxide. The boron is then selectively removed using an acid etching process to form the porous glass film, which is an excellent substrate material for oligonucleotide and other synthesis processes. In the second preferred process, polymer film, such as cross-linked polystyrene, is formed. A solution containing linear polystyrene and UV activated cross-link reagents is injected into and then drained from a microfluidic array device leaving a thin-film coating on the interior surface of the device. The device, which contains opaque masks 564 to define the reaction chamber regions, is next exposed to UV light so as to activate crosslinks between the linear polystyrene chains in the reaction chamber regions. This is followed by a solvent wash to remove non-crosslinked polystyrene, leaving the crosslinked polystyrene only in the reaction chamber regions as shown in FIG. 5D. Crosslinked polystyrene is also an excellent substrate material for oligonucleotide and other synthesis processes.

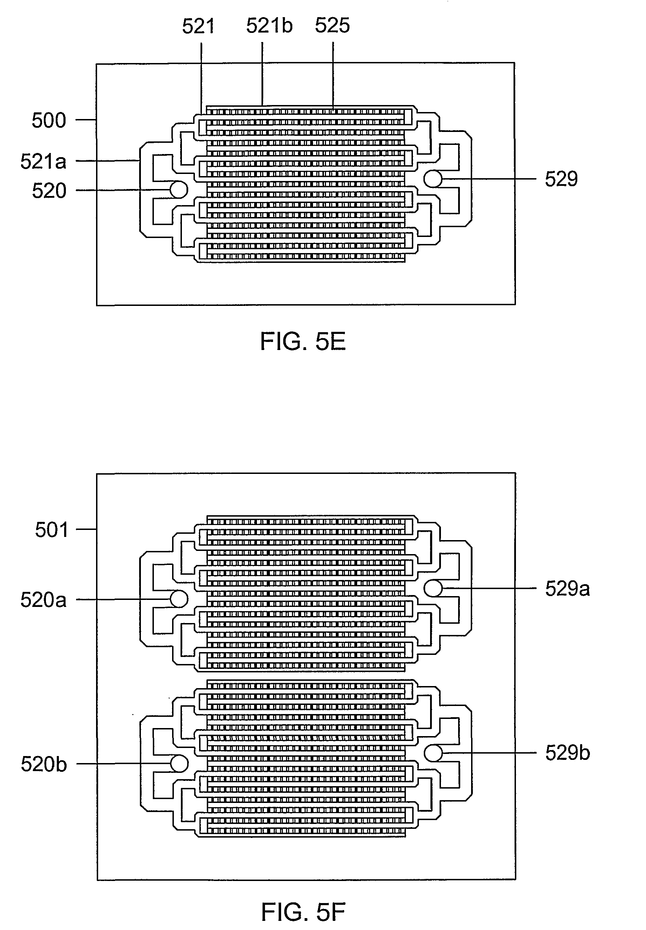

FIG. 5E illustrates the first preferred embodiment of the present invention of a microfluidic array device chip **500.** Binary fluidic distributors **521a** are used to evenly distribute fluid from inlet port **520** into fluid channels **521.** It is preferred to have the same width for all the fluid channels **521** except the side fluid channels **521b,** which are preferably narrower than the middle fluid channels **521** so as to compensate for the reduced volume flow rate in the side fluid channels **521b.** In general, the cross section area of fluid channel **521** is preferably significantly larger than that of a reaction chamber **525** (FIG. 5C) in order to achieve uniform flow across all the reaction chambers **525** along the fluid channel **521.** The cross-section area ratio is preferably between 10 to 10,000. The ratio is more preferably between 100 to 10,000. The ratio is even more preferably between 1,000 to 10,000. On the other hand, one may want to choose a reasonably small cross-section area ratio in order to maximize the use of chip surface area for reaction chambers **525.**

For multiple-sample assay applications, more than one microfluidic array devices can be put on a single chip **501.** FIG. 5F illustrates a chip **501** containing two microfluidic array devices. This type of multiple assay chips may find use in diagnostic applications in clinical laboratories as well as high-throughput screen applications in industrial and research laboratories.

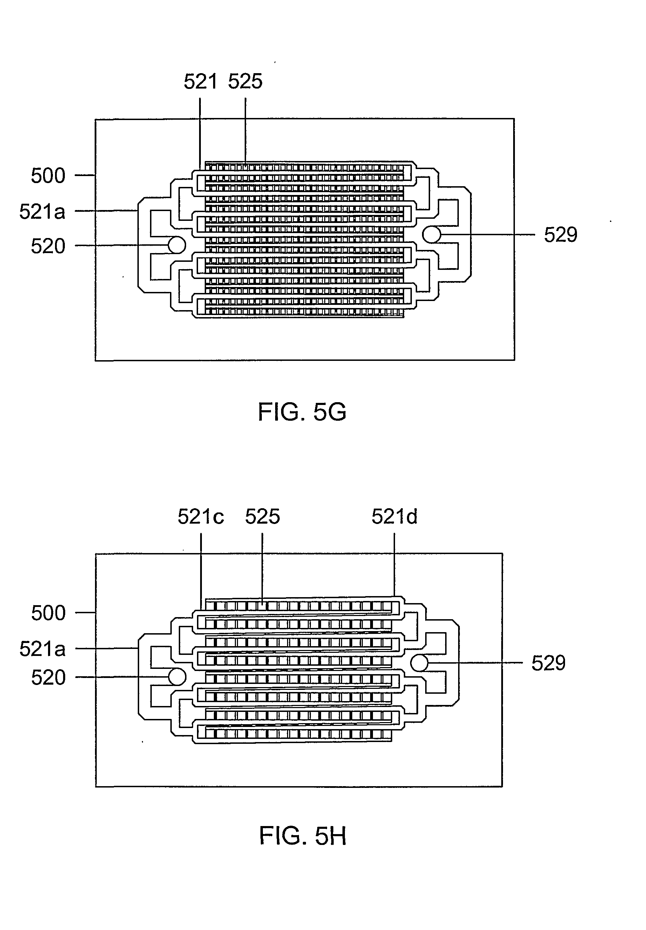

A fluid channel may not have to be straight with a uniform width along its path. FIG. 5G illustrates a second preferred embodiment microfluidic array device of this invention having tapered fluid channels **521.** The sidewall of the taper channels **525** may not have to be straight along the channel. When the taper shape is properly designed, a uniform flow rate can be achieved across all reaction chambers 525 along the fluid channel 521. Suitable fluid channel shapes for producing desirable flow profiles across the reaction chambers along the channels can be derived by those skilled in the art using fluid dynamic simulation methods. Commercial computational fluidic dynamic software packages, such as FLUENT from Fluent Inc., New Hampshire, USA and CFD-ACE from CFD Research Corporation, Alabama, USA, are available and can be used for deriving the channel shapes.

The third preferred fluid channel design is shown in FIG. 5H. Each pair of inlet and outlet fluid channels **521c** and **521d** facilitates the fluid flow of only one column of reaction chambers **525** along the channels instead of two columns of reaction chambers **525** as shown in FIG. 5G. The advantage of this fluid channel design is the simplified fluidic flow in the fluid channels and the elimination of the possibility of cross mixing between adjacent reaction chambers across the commonly shared fluid channels.

The maximum number of cells is not particularly limited. The preferred number of reaction cells on each chip of the present invention is in the range of, for example, 10 to 1,000,000 depending on the desired application of the chip, the reaction chamber size, and the chip size. More preferred is the rage of 100 to 100,000. Even more preferred is the range of 900 to 10,000. Preferably, there are at least two cells, and more preferably, at least 10 cells. Even more preferably, there are at least 100 cells. And even more preferably, there are at least 1,000 cells, and even more preferably, there are at least 10,000 cells.

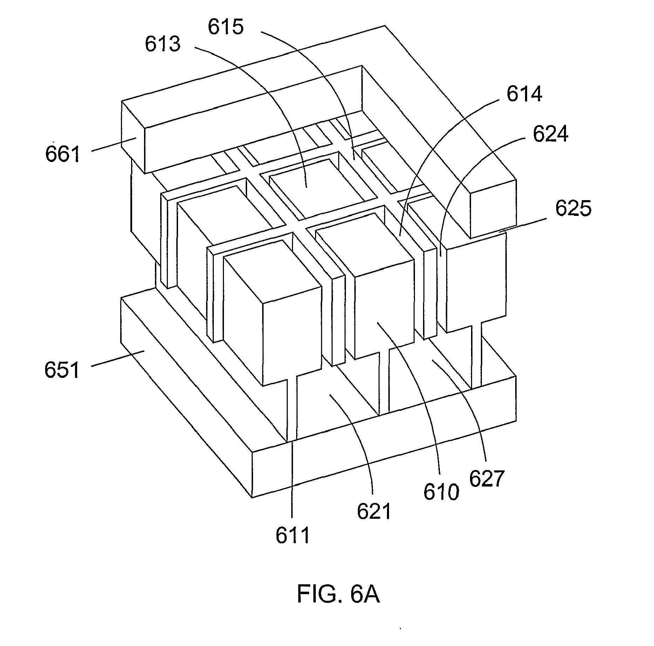

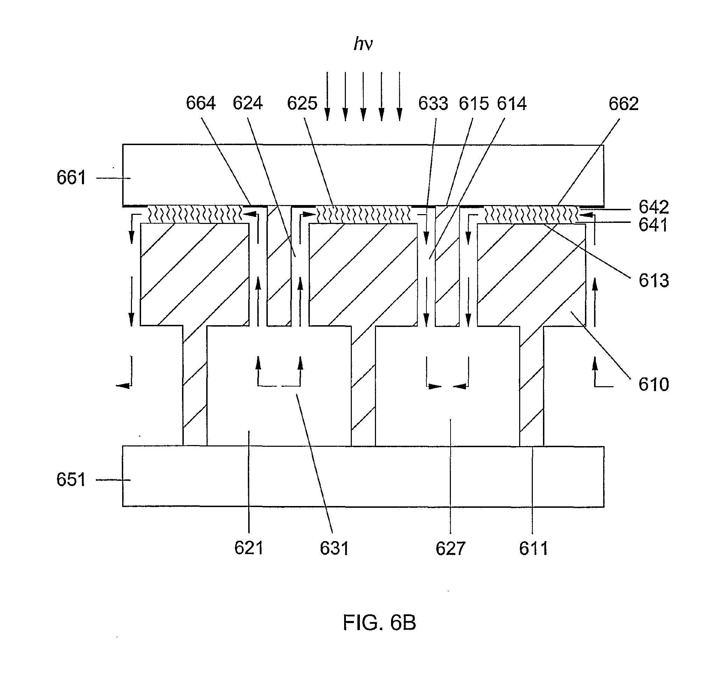

FIG. 6A illustrates an exploded perspective view of a flowthrough multi-cell reactor device, another embodiment of the one-level device configuration shown in FIG. 2C. FIG. 6B schematically illustrates the cross-section of the device shown in FIG. 6A. The back plate **651** and the window plate **661** are bonded with the microfluidic template **610** at the bonding areas **611** and **615** of the microfluidic template **610.** Inlet channel **621** and outlet channel **627** are located between the back plate **651** and microfluidic template **610.** Light exposure and photogenerated-reagent-involved (PGRI) chemical/biochemical reaction are performed in a reaction chamber or cell **625,** formed between the window plate **661** and the microfluidic template **610.** Shadow mask **664** is incorporated into this device design to optically define the reaction chamber **625** on the window plate **661.** This reactor configuration allows the window side of the microfluidic template **610** fully utilized for implementing reaction chamber **625** and is particularly useful for high-density assay applications.

During a reaction involving the use of photogenerated reagents, a feed solution **631** containing photogenerated reagent precursor flows from an inlet channel **621** through an inlet duct **624** into a reaction chamber or cell **625.** When the reaction chamber is illuminated, at least one active reagent is produced, which then reacts with immobilized molecules **641,** and **642** on the top surface **613** of the fluidic template **610** and the inner surface **622** of the window plate **661,** respectively. The effluent **633** flows through an outlet duct **614** into the outlet channel **627.**

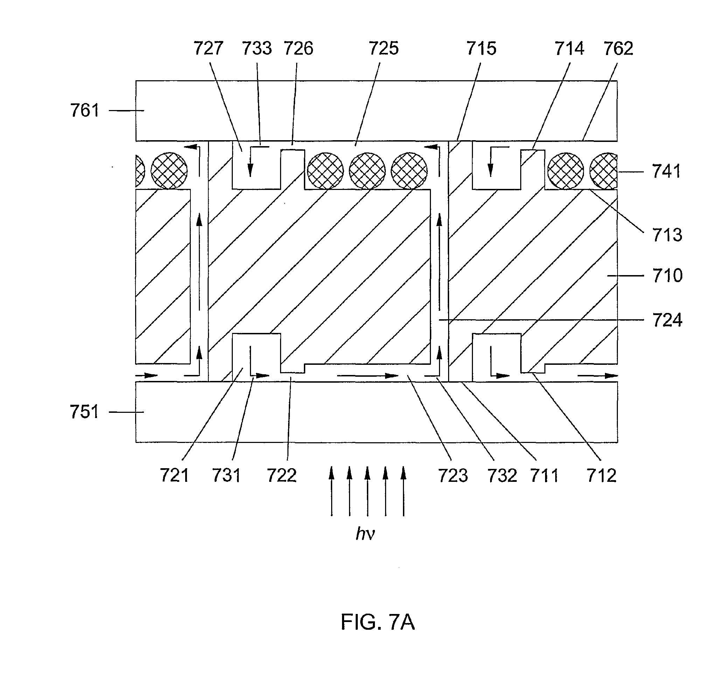

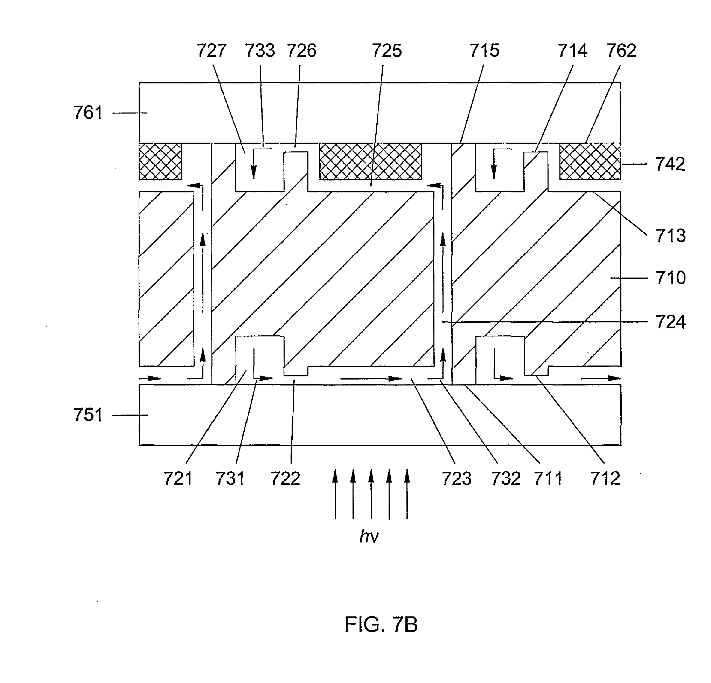

FIG. 7A schematically illustrates a variation of a flowthrough multi-cell reactor with reaction chambers containing beads in which solid-phase chemical reactions take place, another embodiment of the two-level device configuration shown in FIG. 2B. The beads **741** are made of materials including, but not limited to, CPG (controlled pore glasses), cross-linked polystyrene, and various resins that are used for solid-phase synthesis and analysis that have been extensively discussed in "A Practical Guide to Combinatorial Chemistry", edited by Czarnik et al., American Chemical Society, 1997. In one aspect of the present invention, the chemical compounds formed in or on the beads 741 are used for assay applications. The porous or three-dimensional structure of the beads supports high loading of the chemical compounds and therefore, leads to high sensitivity of the assay. Another embodiment of the present invention involving high loading substrate is shown in FIG. 7B. Resin pads **742** are used in place of beads.

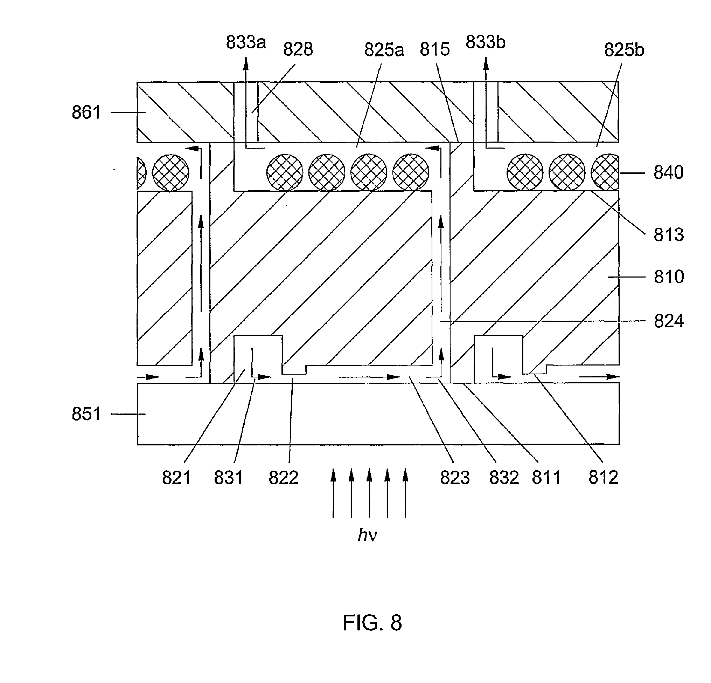

One aspect of the present invention involves single-inlet-multiple-outlet reactor system shown in FIG. 2D. A device embodiment of the reactor system is shown in FIG. 8. Chemical reagents/solvents flow through a reaction cell from inlet channel **821,** to an illumination chamber **823,** to a connection channel **824,** to a reaction chamber **825a,** and exit through an outlet channel **833a.** Chemical reactions take place on the surface of beads **840.** One exemplary application of this reactor device is the parallel synthesis of a plurality of oligonucleotides. Individual oligonucleotide sequences are synthesized on the beads **840** in the individual reaction chambers **825a, 825b,** and others. The product oligonucleotides are collected at outlet channels **833a, 833b,** and others.

With the teaching given above, it is not difficult for those skilled in the art to construct devices implementing the one-level device configuration for single-inlet-multiple-outlet reactor system shown in FIG. 2D

### Device Operation

In a preferred embodiment of the present invention, a device configuration shown in FIG. 3C is used and an array of oligonucleotides for hybridization assay applications is synthesized. The microfluidic template **310** is made of silicon. The first window plate **351** and the second window plate **361** are made of glass. The top surface **313** of the microfluidic template **310** is coated with silicon dioxide. The inner surface areas of the microfluidic device is first derivatised with linker molecules, such as N-(3-triethoxysilylpropyl)-4-hydroxybutyramide (obtainable from Gelest Inc., Tullytown, PA 19007, USA) so that the hydroxyl containing linker molecules are attached to the silicon dioxide and glass surfaces. The derivitization of various solid surfaces is well know to those skilled in the art (Beier et al, in Nucleic Acids Research, 27, 1970, (1999), and references quoted therein). A DMT (4,4'-dimethoxytrityl)-protected spacer phosphoramidite, such as Spacer Phosphoramidite 9 supplied by Glen Research, Sterling, VG 20164, USA, is injected into the reactor and is coupled to the linker molecules. It is well know that the use of the spacer is advantageous for hybridization application of the assay (Southern et al. in Nature Genetics Supplement, 21, 5, (1999)). Photogenerated-acid precursor (PGAP), such as an onium salt SSb (from Secant chemicals Inc., MA 01475, USA) in CH₂Cl₂, is injected into the reactor. While keeping a steady flow of PGAP, a first predetermined group of illumination chambers **325** is illuminated so that photogenerated acid (PGA) is generated and the detritylation (removal of DMT protection groups) takes place in the corresponding reaction cells, which consists of an illumination chamber **323,** a connection channel **324,** and a reaction chamber **325.** A first DMT (4,4'-dimethoxytrityl)-protected phosphoramidite monomer, choosing from dA, dC, dG, and dT (obtainable from Glen Research, Sterling, VG 20164, USA), is injected into the reactor so that the first phosphoramidite monomer is coupled to the spacer in the illuminated reaction cells. No coupling reaction takes place the un-illuminated reaction cells because the spacer molecules in these cells are still protected by DMT groups. The synthesis reaction is preceded with capping and oxidation reactions, which are well known to those skilled in the art of oligonucleotide synthesis (Gait et al, in "Oligonucleotide Synthesis: a Practical Approach", Oxford, 1984). A second predetermined group of illumination chambers are then illuminated followed by the coupling of the second phosphoramidite monomer. The process proceeds until oligonucleotides of all predetermined sequences are formed in all predetermined reaction cells.

Illumination of predetermined illumination chambers can be performed using various well-known methods including, not limited to, digital-micromirror-device-based light projection, photomask-based projection, and laser scanning. The wavelength of the illumining light should match the excitation wavelength of PGAP. For example, when SSb PGAP is used, a light source with a center wavelength about 366 nm is preferred. Details on the selection of PGAP, illumination conditions, and methods of illumination are described in, for example, Gao et al. WO09941007A2, which is incorporated by reference.

One aspect of the present invention involves confining synthesis reactions in designated areas. For example, in the assay application of the reactor device shown in FIG. 3C the immobilized molecules **341** and **342** are used as probes, which are preferably synthesized only in the areas under the shadow mask **364.** In a preferred embodiment of the present invention, linker molecules are first immobilized to the internal surface of the reactor device and a photolabile-group-protected phosphoramidite, such as 5'-[2-(2-nitrophenyl)-propyloxycarbonyl]-thymidine (NPPOC, Beier el al., Nucleic Acids Res. 28, e11 (2000)), is coupled to the linkers forming photolabile-group-protected linker intermediates. All the illumination chambers **323** are then illuminated to remove the 2-(2-nitrophenyl)-propyloxycarbonyl photolabile protection groups from the linker intermediates on the internal surfaces of the illumination chambers **323.** The deported linker intermediates are then capped with a capping reagent (obtainable from Glen Research, Sterling, VG 20164, USA) so as to prevent any further growth of oligonucleotides in the illumination chamber. Next, the reaction chambers **325** are flush-illuminated through a glass second window **361** to remove the photolabile protection groups from the linker intermediates on the inner surfaces **362** of the second window **361** and the top surface **313** of the fluidic template **310.** These surface areas, therefore, become available for further growth of oligonucleotides. The internal surface areas of the connection channels **324** are not exposed to light and the photolabile protection groups on the linker intermediates block any chemical reactions on the channel surface areas during a nucleotide synthesis.

Special cares for the removal of gas bubbles from reagent delivery manifold should be taken, especially when a flowthrough reactor system contains small sized reaction cells. Various methods of gas removal from liquid phase media are available and are well known to those skilled in the art. The methods include, but not limited to, use of degassing membranes, helium sparging, and in-line bubble traps. Various gas removal devices are available from commercial companies such as Alltech Associates Inc., Deerfield, IL 60015, USA.

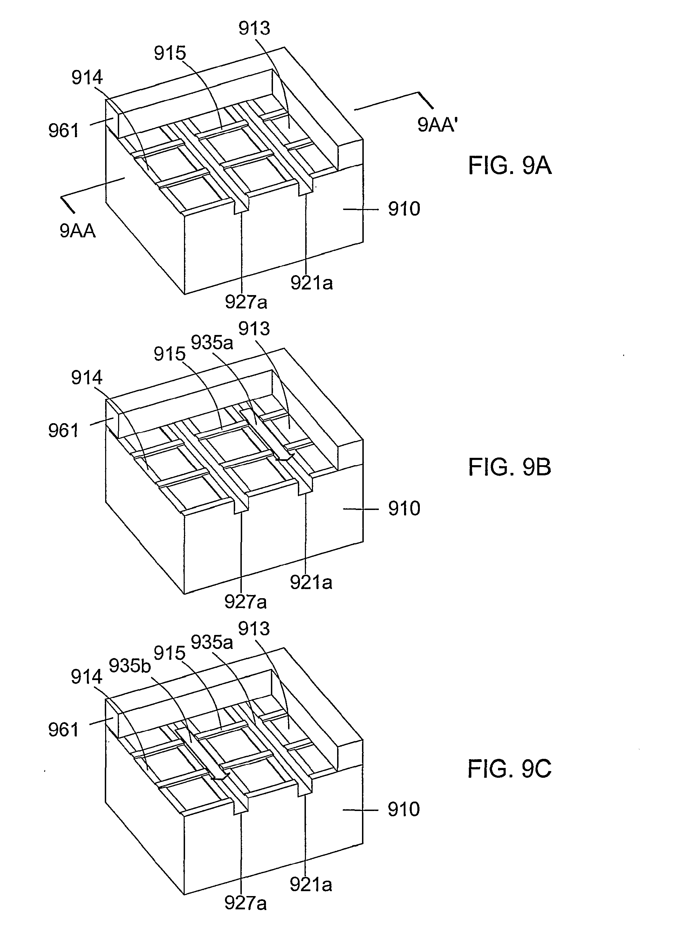

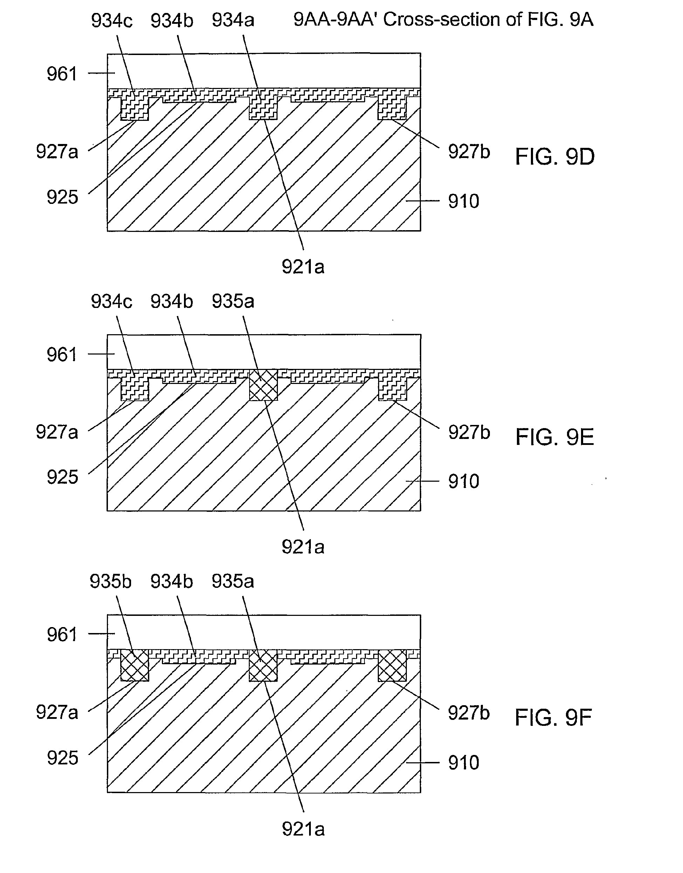

Another use of the microfluidic array devices of this invention is to perform parallel assays that require the physical isolation of individual reaction cells. The first preferred operation method is illustrated in FIG. 9A through FIG. 9F. The device is first filled with the first fluid **934a, 934b,** and **934c** as shown in FIG. 9D. The first fluid is the one that will remain in the reaction chambers **925** after the cell isolation. If the first fluid is an aqueous solution, the internal surface of the reaction chambers **925** is preferably hydrophilic. For example, surface immobilized with oligo DNA molecules are hydrophilic. If the first fluid is a hydrophobic solution, the internal surface of the reaction chambers **925** is preferably hydrophobic. The second fluid **935a,** which is non-mixable with the first fluid and is preferably inert, is then injected into the device through the first set of fluid channels **921a** while keeping the second set of fluid channel **927a** and **927b** blocked as illustrated in FIG. 9B. In case of the first fluid being an aqueous solution and the internal surface of the reaction chambers **925** being hydrophilic, the second fluid is preferably a hydrophobic liquid such as liquid paraffin, silicon oil, or mineral oil. Due to surface tension effect and the pressure resistance the second fluid **935a** only replaces the first fluid **934a** in the first set of fluid channels **927a** and does not replace the first fluid **934b** in the reaction chambers **925** as shown in FIG. 9E. The third fluid **935b,** which is preferably the same liquid material as the second fluid **935a,** is then injected into the device through the second set of fluid channels **927a** and **927b** while keeping the first set of fluid channels **921a** blocked as illustrated in FIG. 9C. As result, the first fluid **934c** in the second set of channels **927a** and **927b** is replace by the third fluid **935b** completing the isolation of the first fluid **934b** in the reaction chambers **925,** as shown in FIG. 9F. The microfluidic array devices of this invention and the isolation method described in this section can be used to perform various biological, biochemical and chemical assays that have been developed on micro-titer or microwell plate platforms. The main advantages of the present invention include significantly reduced sample size, significantly increased assay density (number of assays performed in each experiment), and reduction of cost.

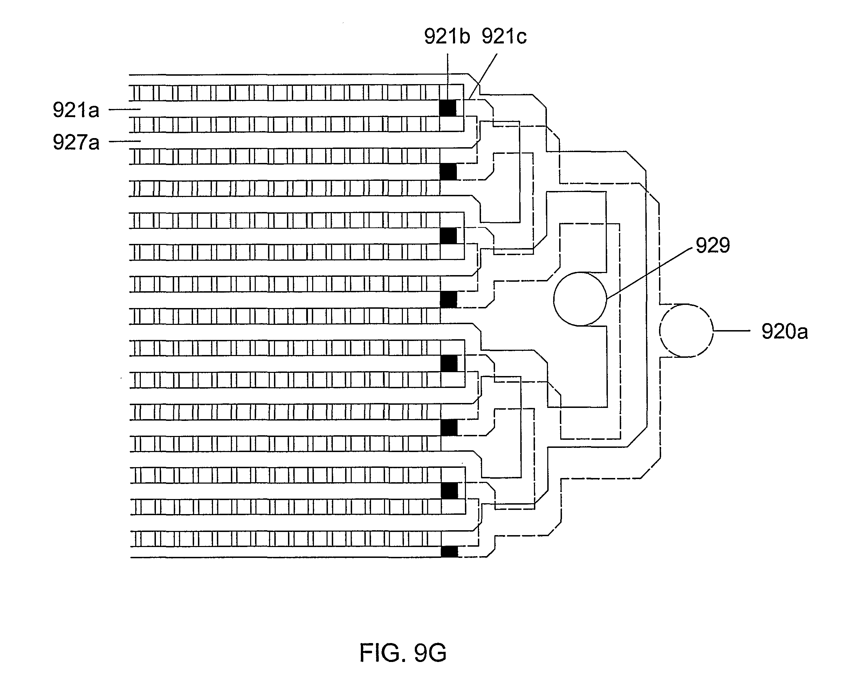

To utilize the above isolation method, fluid distribution channels are preferably arranged differently from those shown in FIG. 5E through FIG. 5H. An important feature is a pass at the end of each fluid channel so that a fluid can flow through the channel without having to pass through reaction chambers. For example, a preferred embodiment is shown in FIG. 9G. In this embodiment, fluid channels **921a** and **927a** are located at the front or the first side of a fluidic template. At the end of each fluid channel **921a** there is a through-hole **921b** that allows fluid to flow to the backside or the second side of the fluidic template. On the backside of the fluidic template, binary fluid distribution channels **921c** and outlet port **920a** are implemented, as drawn with dash lines in FIG. 9G. Those skilled in the art of microfluidics should be able to following the teaching of this invention to design and/or construct various variations of the fluidic structures to accomplish the isolation method.

The disclosures of Gao et al., J. Am. Chem. Soc., 120, 12698-12699 (1998) and WO 09941007A2 are hereby incorporated by reference. Methods and apparatuses of the present invention are useful for preparing and assaying very-large-scale arrays of DNA and RNA oligonucleotides, peptides, oligosaccharides, phospholipids and other biopolymers and biological samples on a substrate surface. Light-directed on-chip parallel synthesis can be used in the fabrication of very-large-scale oligonucleotide arrays with up to one million sequences on a single chip.

The photo-reagent precursor can be, different types of compounds including for example, diazonium salts, perhalomethyltriazines, halobisphenyl A, o-nitrobenzaldehyde, sulfonates, imidylsulfonyl esters, diaryliodonium salts, sulfonium salts, diazosulfonate, diarylsulfones, 1,2-diazoketones, diazoketones, arylazide derivatives, benzocarbonates or carbamates, dimethoxybenzoin yl carbonates or carbamates, o-nitrobenzyloxycarbonates or carbamates, nitrobenzenesulphenyl, and o-nitroanilines.

The invention is further described by the following EXAMPLES, which are provided for illustrative purposes only and are not intended nor should they be construed as limiting the invention in any manner. Those skilled in the art will appreciate those variations on the following EXAMPLES can be made without deviating from the spirit or scope of the invention.

### EXAMPLE I

### Microfluidic Device Fabrication

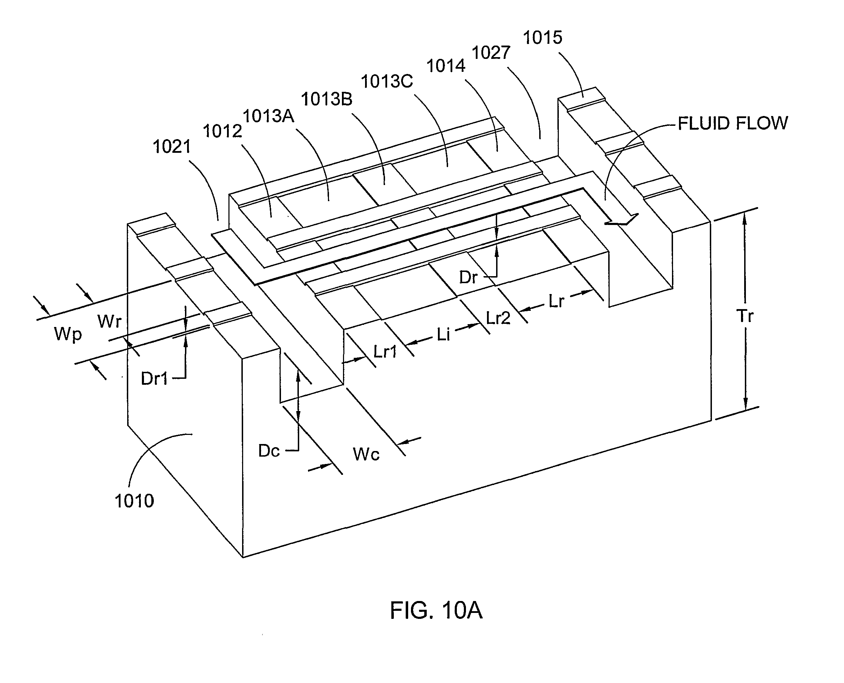

Microfluidic reactor devices having a device structure shown in FIG. 10A are fabricated using silicon-micro-machining processes. Si (100) substrates having a thickness Tᵣ between 450 to 500 µm are used. A microfluidic template **1010** comprises inlet channel **1021** and outlet channel **1027,** inlet restriction ridge **1012,** exposure chamber **1013A,** dividing ridge **1013B,** reaction chamber **1013C,** and outlet restriction ridge **1014.** An enclosed microfluidic reactor device is formed by bonding the microfluidic template **1010** with a glass plate (not shown in the figure) at the bonding areas **1015.** The direction of the fluid flow is shown in the figure. In this device, the inlet channels **1021** and outlet channels have the same dimensions of depth D_{c} of about 150 µm and width W_{c} of 90 µm. The inlet restriction ridge **1012,** the dividing ridge 1013B, and the outlet restriction ridge **1014** have the same width Lᵣ₁ of 30 µm and gap Dᵣ₁ of about 12 µm. The illumination chamber **1013A** has a length Lᵢ of 120 µm and depth Dᵣ of about 16 µm. The reaction chamber **1013C** has a length Lᵣ of 120 µm and depth Dᵣ of about 16 µm.

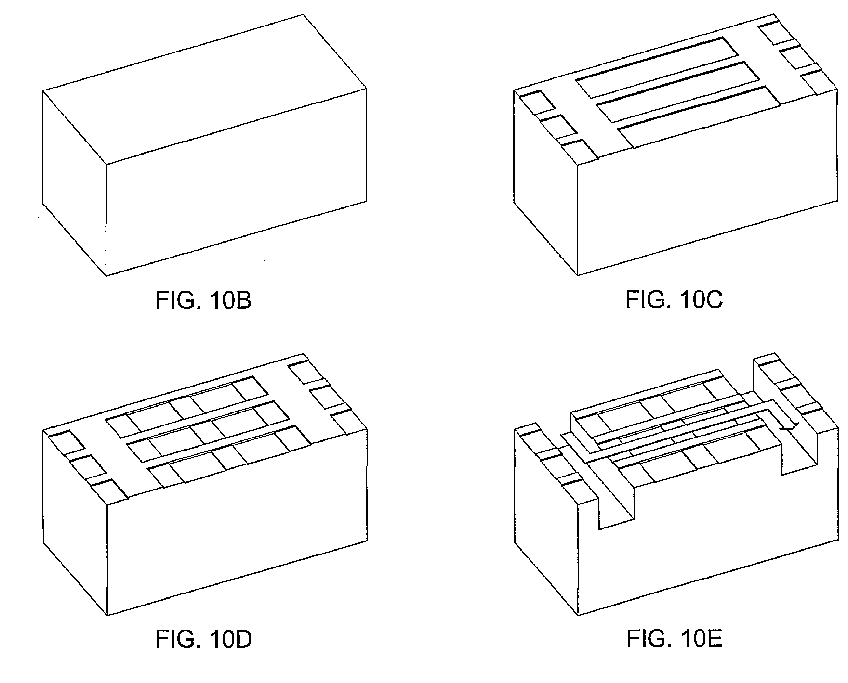

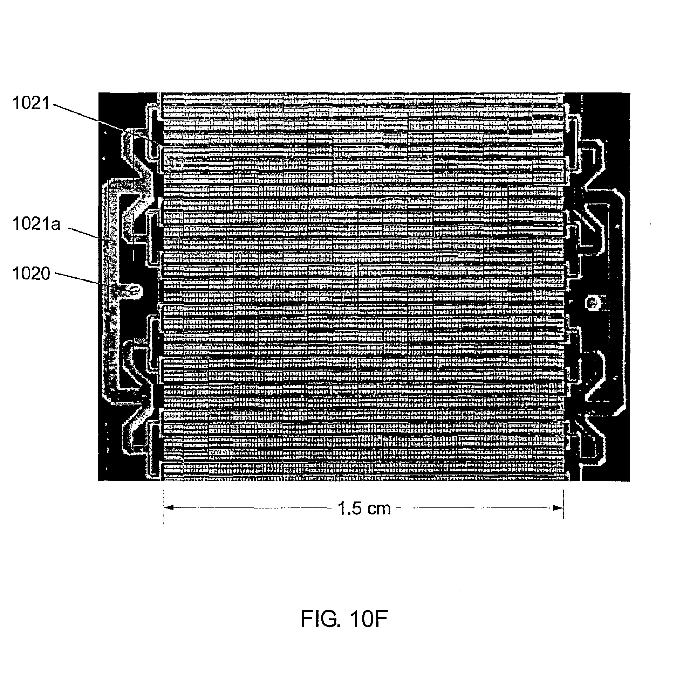

The fabrication starts from a flat Si (100) wafer shown in FIG. 10B. A photoresist (e.g. AZ 4620 from Shipley Company, Marlborough, MA 01752, USA) is spin coated on the surface of the wafer. The photoresist film is then dried, exposed and developed using a photolithographic method. The wafer is then etched using Inductively Coupled Plasma (ICP) silicon etcher (from Surface Technology Systems Limited, UK) for about 12 µm. Then, the photoresist film is stripped. The resulted structure is shown in FIG. 10C. The silicon substrate is spin-coated with the second layer of photoresist. The photoresist is dried, exposed, and developed. The silicon substrate is then etched with the ICP silicon etcher for about 4 µm and the photoresist is stripped, resulting in the structure shown in FIG. 10D. Next, the surface of the silicon structure is spin-coated with the third layer photoresist. The photoresist is dried, exposed, and developed. The silicon substrate is then etched with the ICP silicon etcher for about 150 µm and the photoresist is stripped. The resulting microfluidic template is shown in FIG. 10E. A thin layer (about 50 to 200 Å) of SiO₂ is then coated on the surface of the structure using a Chemical Vapor Deposition (CVD) method. In the final step, the silicon microfluidic template is bonded with a Pyrex glass wafer (Coming 7740 from Corning Incorporated, Coming, NY 14831) using anodic bonding method (Wafer Bonding System from EV Group Inc., Phoenix, AZ 85034, USA). The photograph of a finished microfluidic array device is shown in FIG. 10F.

### EXAMPLE II

### Oligonucleotide Array Synthesis

The microfluidic reactor device made in EXAMPLE I was used for producing oligonucleotide arrays. Chemical reagents were delivered to the reactor by a HPLC pump, a DNA synthesizer (Expedite 8909, manufactured by PE Biosystems, Foster City, CA 94404, USA) or a Brinkman syringe dispenser (Brinkmann Instruments, Inc., Westbury, NY 11590, USA), each equipped with an inline filter placed before the inlet of the reactor. The microfluidic reactor device was first washed using 10 ml 95% ethanol and then derivatized using a 1% solution of N-(3-Triethoxy-silylpropyl)-4-hydroxybutyramide (linker) in 95% ethanol at a flow rate 0.15 ml/min. After 12 hours, the flow rate was increased to 3 ml/min for 4 hours. The microfluidic reactor device was then washed with 10 ml 95% ethanol at a flow rate of 3 ml/min and dried with N₂ gas. The device was placed in a chamber at about 60 °C and N₂ was circulated inside the device for 4 hours to cure the linker layer.

Deoxyoligo-TT (thymine nucleotide dimer) DNA synthesis was carried out using standard phosphoramidite chemistry and reagents (synthesis protocol is provided by in the Operation Manual of Expedite 8909 DNA Synthesizer). At the end of the TT synthesis step the whole internal surface, including the internal surface of the reaction and radiation and reaction chambers (**1013A** and **1013C** in FIG. 10A), of the microfluidic reactor device is covered with TT nucleotide dimers. The end of the TT dimer is protected with acid labile DMT group.

PGA involved phosphoramidite synthesis was then performed under various radiation conditions to demonstrate the activation of PGA for DMT deprotection reaction. The PGAP involved chemical reactions are described by Gao et al. in WO09941007A2. In this example, the PGAP used was a two-component system consisting of 3% Rhodorsyl (obtained from Secant chemicals Inc., MA 01475, USA) and 2 equivalent Cholo (obtained from Aldrich, Milwaukee, WI 53233, USA) in CH₂Cl₂. The flow rate for the PGA solution was 0.05 ml/min. A computer controlled Digital Light Projector (DLP) is used to generate photolithographic patterns for activating photochemical reactions in predetermined reaction cells in the microfluidic reactor device. The construction and operation of DLP are described by Gao et al. in WO09941007A2. A 500 W mercury lamp (from Oriel Corporation, Stratford, CT 06497, USA) was used as the light source and a dichroic filter was used to allow only the wavelength between 350 and 450 nm to be applied. Among predetermined illumination chambers (**1013A** in FIG. 10A) the length of irradiation was varied from 1 second to 20 seconds and the irradiation intensity was varied from 10% to 100% of the full intensity of 28 mW/cm². After the light exposure, additional 0.5 ml un-activated PGAP solution was injected in the microfluidic reactor device to flush residue acids out of the reactor. Then the reactor was washed with 4 ml 20% pyridine in acetonitrile. A fluorescein phosphoramidite coupling reaction is then performed by injecting a solution mixture of 1:2 fluorescein-phosphoramidite:T-phosphoramidite into the reactor device. A thorough wash was carried out with the injection of 20 ml ethanol. The fluorescein moiety was activated by injecting 5 ml of 1:1 ethylene-diamine:anhydrous-ethanol at a 1 ml/min flow rate. The microfluidic reactor device was washed with ethanol and dried with N₂.

Fluorescence imaging was performed under 495 nm light excitation and recorded using a cooled CCD camera (from Apogee Instruments, Inc., Tucson, Arizona 85715, USA) with a band-pass filter centered at 525nm (from Omega Optical, Inc., Brattleboro, Vermont 05302, USA).

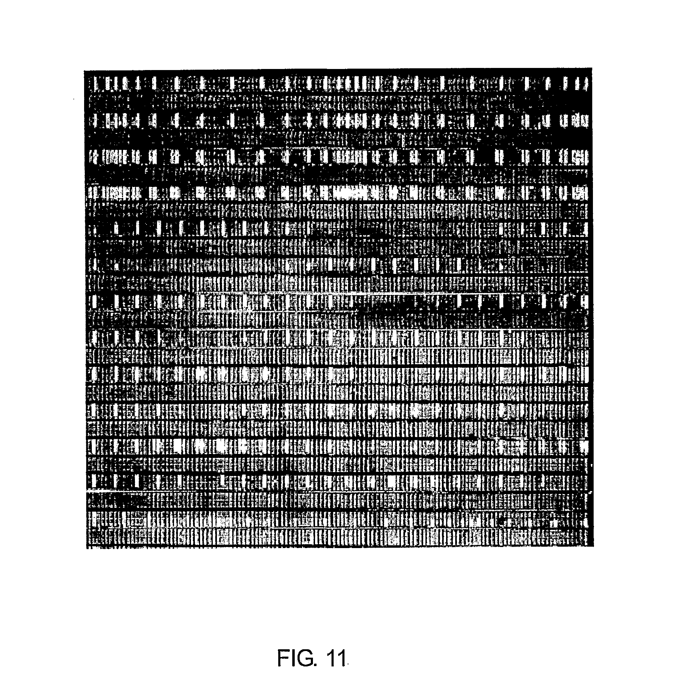

FIG. 11 shows the fluorescence image of the microfluidic reactor device. The degree of DMT deprotection reaction in each illumination/reaction chamber (**1013A** and **1013C** in FIG. 10A) is assayed by the fluorescein-phosphoramidite coupling reaction, which is measured by the fluorescence intensity from the illumination/reaction chamber.

### EXAMPLE III

### Hybridization of Oligonucleotide Array

A microfluidic reactor device was made using the fabrication procedures described in EXAMPLE I. The device was derivatized using the procedures described in EXAMPLE II. Oligonucleotide probes of predetermined sequences were synthesized by the procedures described in EXAMPLE II. The sequences of the probes were 3'TATGTAGCCTCGGTC **1242a** and 3'AGTGGTGGAACTTGACTGCGGCGTCTT **1242b.**

Target nucleosides of 15 nucleotides long and complementary to the 5' ends of the probe sequences were chemically synthesized using standard phosphoramidite chemistry on a DNA synthesizer (Expedite 8909, manufactured by PE Biosystems, Foster City, CA 94404, USA). The targets were labeled with fluorescein at the 5' end. Hybridization was performed using 50 to 100 n molars of the targets in 100 micro liters of 6XSSPE buffer solution (0.9 M NaCl, 60 mM Na₂HPO₄-NaH₂PO₄ (pH 7.2), and 6 mM EDTA) at room temperature for 0.5 to 1.0 hours followed by a wash using the buffer solution. A micropore-tube peristaltic pump was used to facilitate the solution circulation through the microfluidic array device during the hybridization and wash.

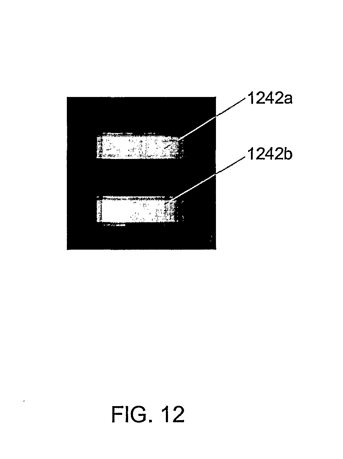

Fluorescence imaging was performed under 495 nm light excitation and recorded using a cooled CCD camera (from Apogee Instruments, Inc., Tucson, Arizona 85715, USA) with a band-pass filter centered at 525nm (from Omega Optical, Inc., Brattleboro, Vermont 05302, USA). FIG. 12 shows the fluorescence image of the microfluidic array device after the hybridization. The five reaction cells shown in the figure include 3'TATGTAGCCTCGGTC **1242a,** 3'AGTGGTGGAACTTGACTGCGGCGTCTT **1242b** and three blank cells.

These examples are non-limiting. They illustrate but do not represent or define the limits of the invention(s).

The following numbered paragraphs are not claims, but represent specific aspects and embodiments of the invention:
1. A microfluidic reactor comprising:
   a plurality of flow-through reaction cells for parallel chemical reactions, each reaction cell comprising:
      i. at least one illumination chamber, and
      ii. at least one reaction chamber,
   wherein the illumination chamber and the reaction chamber are in flow communication and are spatially separated in the reaction cell.
2. A microfluidic reactor according to claim 1, wherein the reactor comprises at least 10 reaction cells.
3. A microfluidic reactor according to claim 1, wherein the reactor comprises at least 100 reaction cells.
4. A microfluidic reactor according to claim 1, wherein the reactor comprises at least 1,000 reaction cells.
5. A microfluidic reactor according to claim 1, wherein the reactor comprises at least 10,000 reaction cells.
6. A microfluidic reactor according to claim 1, wherein the reactor comprises 900 to 10,000 reaction cells.
7. A microfluidic reactor according to claim 1, wherein the reaction cells are adapted for use of in situ generated chemical reagents which are generated in the illumination chamber.
8. A microfluidic reactor according to claim 1, wherein the reactor comprises a silicon microfluidic template.
9. A microfluidic reactor according to claim 1, wherein the reactor comprises a plastic microfluidic template.
10. A microfluidic reactor according to claim 1, wherein a distance between reaction cells which are adjacent to each other is 10 to 5,000 microns.
11. A microfluidic reactor according to claim 1, wherein a distance between reaction cells which are adjacent to each other is 10 to 2,000 microns.
12. A microfluidic reactor according to claim 1, wherein a distance between reaction cells which are adjacent to each other is 10 to 500 microns.
13. A microfluidic reactor according to claim 1, wherein a distance between reaction cells which are adjacent to each other is 10 to 200 microns.
14. A microfluidic reactor according to claim 1, wherein a distance between reaction cells which are adjacent to each other is larger than 5,000 microns.
15. A microfluidic reactor according to claim 1, wherein the reactor comprises a microfluidic template and at least one window plate.
16. A microfluidic reactor according to claim 1, wherein the reactor further comprises at least one shadow mask.
17. A microfluidic reactor according to claim 1, wherein the reactor is adapted to avoid chemical intermixing between the reaction cells.
18. A microfluidic reactor according to claim 1, wherein the reactor further comprises an inlet channel and an inlet restriction gap connected to the illumination chamber, and an outlet channel and an outlet restriction gap connected to the illumination chamber.
19. A microfluidic reactor according to claim 1, wherein the reactor further comprises inlet channels and inlet restriction gaps in fluid communication with the illumination chambers of the reaction cells, and wherein the reactor further comprises outlet channels and outlet restriction gaps in fluid communication with the reaction chambers of the reaction cells, and wherein illumination chambers and reaction chambers of the reaction cells are connected by connection channels.
20. A microfluidic reactor according to claim 1, wherein the reactor further comprises one common inlet channel, branch inlet channels, branch outlet channels, and one common outlet channel.
21. A microfluidic reactor according to claim 1, wherein the reactor further comprises immobilized molecules in the reaction chamber.
22. A microfluidic reactor according to claim 21, wherein the immobilized molecules are biopolymers.
23. A microfluidic reactor according to claim 21, wherein the immobilized molecules are immobilized with use of linker molecules.
24. A microfluidic reactor according to claim 21, wherein the immobilized molecules are selected from the group consisting of DNA, RNA, DNA oligonucleotides, RNA oligonucleotides, peptides, oligosaccharides, and phospholipids.
25. A microfluidic reactor according to claim 21, wherein the immobilized molecules are oligonucleotides.
26. A microfluidic reactor according to claim 1, wherein the reactor further comprises DNA, RNA, DNA oligonucleotides, RNA oligonucleotides, peptides, oligosaccharides, phospholipids, or combinations thereof adsorbed to the reaction chamber.
27. A microfluidic reactor according to claim 1, wherein the reactor further comprises immobilized molecules in a double-layer configuration in the reaction chamber.
28. A microfluidic reactor according to claim 1, wherein the reactor further comprises a three-dimensional attachment of immobilized molecules in the reaction chamber.
29. A microfluidic reactor according to claim 1, further comprising porous films in the reaction chamber.
30. A microfluidic reactor according to claim 29, wherein the porous films are porous glass films or porous polymer films.
31. A microfluidic reactor according to claim 1, wherein the reaction chambers are in capillary form.
32. A microfluidic reactor according to claim 31, wherein the reaction chambers in capillary form have diameters of 0.05 micrometers to 500 micrometers.
33. A microfluidic reactor according to claim 31, wherein the reaction chambers in capillary form have diameters of 0.1 micrometers to 100 micrometers.
34. A microfluidic reactor according to claim 1, wherein the reactor is in the form of an array device chip comprising fluid channels to distribute fluid to the plurality of reaction cells for parallel chemical reaction.
35. A microfluidic reactor according to claim 34, wherein the fluid channels have a first cross sectional area, the reaction cells have a second cross sectional area which is smaller than the first cross sectional area, and the ratio between the first and second cross sectional areas is 10 to 10,000.
36. A microfluidic reactor according to claim 34, wherein the fluid channels have a first cross sectional area, the reaction cells have a second cross sectional area which is smaller than the first cross sectional area, and the ratio between the first and second cross sectional areas is 100 to 10,000.
37. A microfluidic reactor according to claim 34, wherein the fluid channels have a first cross sectional area, the reaction cells have a second cross sectional area which is smaller than the first cross sectional area, and the ratio between the first and second cross sectional areas is 1,000 to 10,000.
38. A microfluidic reactor according to claim 34, wherein the fluid channels are tapered.
39. A microfluidic reactor according to claim 38, wherein the tapered fluid channels provide uniform flow rates across reaction cells along the fluid channels.
40. A microfluidic reactor according to claim 1, wherein the reaction chambers contain beads.
41. A microfluidic reactor according to claim 1, wherein the reaction chambers contain resin pads.
42. A microfluidic reactor according to claim 1, wherein the reactor comprises an array of oligonucleotides in the reaction chamber, a microfluidic template made of silicon, and first and second window plates made of glass and attached to the template.
43. A microfluidic reactor according to claim 1, wherein the device comprises an array of oligonucleotides in the reaction chambers, a microfluidic template made of silicon, window plates, a shadow mask, inlet channels and inlet restriction gaps connected to the illumination chambers, outlet channels and outlet restriction gaps connected to the reaction chambers, distribution channels for parallel reactions in the reaction cells, and connection channels to connect illumination and reaction chambers.
44. A microfluidic reactor according to claim 43, wherein the reactor is in the form of an array device chip comprising fluid channels to distribute fluid to the plurality of reaction cells for parallel chemical reactions.
45. A microfluidic reactor according to claim 44, wherein the reactor comprises at least 10 reaction cells.
46. A microfluidic reactor according to claim 45, wherein the oligonucleotides are immobilized with use of linker molecules.
47. A microfluidic reactor according to claim 46, wherein the reaction cells, illumination chambers, and reaction chambers are adapted for use of in situ generated chemical reagents.
48. A chip comprising a plurality of microfluidic reactors according to claim 1.
49. A chip comprising a plurality of microfluidic reactors according to claim 43.
50. A microfluidic reactor comprising a plurality of flow-through photoillumination reaction cells for parallel chemical reactions in fluid communication with at least one inlet channel and at least one outlet channel.
51. A microfluidic reactor according to claim 50, wherein the reactor comprises at least 10 reaction cells.
52. A microfluidic reactor according to claim 50, wherein the reactor comprises at least 100 reaction cells.
53. A microfluidic reactor according to claim 50, wherein the reactor comprises at least 1,000 reaction cells.
54. A microfluidic reactor according to claim 50, wherein the reactor comprises at least 10,000 reaction cells.
55. A microfluidic reactor according to claim 50, wherein the reactor comprises 900 to 10,000 reaction cells.
56. A microfluidic reactor according to claim 50, wherein the reaction cells are adapted for use of in situ generated chemical reagents which are generated in the reaction cell.
57. A microfluidic reactor according to claim 50, wherein the reactor comprises a silicon microfluidic template.
58. A microfluidic reactor according to claim 50, wherein the reactor comprises a plastic microfluidic template.
59. A microfluidic reactor according to claim 50, wherein a distance between reaction cells which are adjacent to each other is 10 to 5,000 microns.
60. A microfluidic reactor according to claim 50, wherein a distance between reaction cells which are adjacent to each other is 10 to 2,000 microns.
61. A microfluidic reactor according to claim 50, wherein a distance between reaction cells which are adjacent to each other is 10 to 500 microns.
62. A microfluidic reactor according to claim 50, wherein a distance between reaction cells which are adjacent to each other is 10 to 200 microns.
63. A microfluidic reactor according to claim 50, wherein a distance between reaction cells which are adjacent to each other is larger than 5,000 microns.
64. A microfluidic reactor according to claim 50, wherein the reactor comprises a microfluidic template and at least one window plate.
65. A microfluidic reactor according to claim 50, wherein the reactor further comprises at least one shadow mask.
66. A microfluidic reactor according to claim 50, wherein the reactor is adapted to avoid chemical intermixing between the reaction cells.
67. A microfluidic reactor according to claim 50, wherein the reactor further comprises inlet restriction gaps and outlet restriction gaps connected to the reaction cells.
68. A microfluidic reactor according to claim 50, wherein the reactor further comprises one common inlet channel, branch inlet channels, branch outlet channels, and one common outlet channel.
69. A microfluidic reactor according to claim 50, wherein the reactor further comprises immobilized molecules in the reaction cell.
70. A microfluidic reactor according to claim 69, wherein the immobilized molecules are biopolymers.
71. A microfluidic reactor according to claim 69, wherein the immobilized molecules are immobilized with use of linker molecules.
72. A microfluidic reactor according to claim 69, wherein the immobilized molecules are selected from the group consisting of DNA, RNA, DNA oligonucleotides, RNA oligonucleotides, peptides, oligosaccharides, and phospholipids.
73. A microfluidic reactor according to claim 69, wherein the immobilized molecules are oligonucleotides.
74. A microfluidic reactor according to claim 50, wherein the reactor further comprises DNA, RNA, DNA oligonucleotides, RNA oligonucleotides, peptides, oligosaccharides, phospholipids, or combinations thereof adsorbed to the reaction cell.
75. A microfluidic reactor according to claim 50, wherein the reactor further comprises immobilized molecules in a double-layer configuration in the reaction cell.
76. A microfluidic reactor according to claim 50, wherein the reactor further comprises a three-dimensional attachment of immobilized molecules in the reaction cell.
77. A microfluidic reactor according to claim 50, further comprising porous films in the reaction cell.
78. A microfluidic reactor according to claim 77, wherein the porous films are porous glass films or porous polymer films.
79. A microfluidic reactor according to claim 50, wherein the reaction cells are in capillary form.
80. A microfluidic reactor according to claim 79, wherein the reaction cells in capillary form have diameters of 0.05 micrometers to 500 micrometers.
81. A microfluidic reactor according to claim 79, wherein the reaction chambers in capillary form have diameters of 0.1 micrometers to 100 micrometers.
82. A microfluidic reactor according to claim 50, wherein the reactor is in the form of an array device chip comprising fluid channels to distribute fluid to the plurality of reaction cells for parallel chemical reactions.
83. A microfluidic reactor according to claim 82, wherein the fluid channels have a first cross sectional area, the reaction cells have a second cross sectional area which is smaller than the first cross sectional area, and the ratio between the first and second cross sectional areas is 10 to 10,000.
84. A microfluidic reactor according to claim 82, wherein the fluid channels have a first cross sectional area, the reaction cells have a second cross sectional area which is smaller than the first cross sectional area, and the ratio between the first and second cross sectional areas is 100 to 10,000.
85. A microfluidic reactor according to claim 82, wherein the fluid channels have a first cross sectional area, the reaction cells have a second cross sectional area which is smaller than the first cross sectional area, and the ratio between the first and second cross sectional areas is 1,000 to 10,000.
86. A microfluidic reactor according to claim 82, wherein the fluid channels are tapered.
87. A microfluidic reactor according to claim 86, wherein the tapered fluid channels provide uniform flow rates across reaction cells along the fluid channels.
88. A microfluidic reactor according to claim 50, wherein the reaction cells contain beads.
89. A microfluidic reactor according to claim 50, wherein the reaction cells contain resin pads.
90. A microfluidic reactor according to claim 50, wherein the reactor comprises an array of oligonucleotides in the reaction cells, a microfluidic template made of silicon, and first and second window plates made of glass bonded to the template.
91. A microfluidic reactor according to claim 50, wherein the device comprises an array of oligonucleotides in the reaction cells, a microfluidic template made of silicon, window plates, a shadow mask, inlet restriction gaps connected to the reaction cells, outlet restriction gaps connected to the reaction cells, and distribution channels to connect the reaction cells for parallel chemical reactions.
92. A microfluidic reactor according to claim 50, wherein the reactor is in the form of an array device chip comprising fluid channels to distribute fluid to the plurality of reaction cells for parallel chemical reactions.
93. A microfluidic reactor according to claim 92, wherein the reactor comprises at least 10 cells.
94. A microfluidic reactor according to claim 91, wherein the oligonucleotides are immobilized with use of linker molecules.
95. A microfluidic reactor according to claim 94, wherein the reaction cells are adapted for use of in situ generated chemical reagents.
96. A microfluidic reactor according to claim 50, wherein the inlet channel and the outlet channel are located on the same side of a microfluidic template.
97. A microfluidic reactor according to claim 50, wherein the reactor comprises one common inlet channel and one common outlet channel.
98. A microfluidic reactor according to claim 50, wherein the reaction cells each comprise an illumination chamber and a reaction chamber which partially overlap each other.
99. A chip comprising a plurality of microfluidic reactors according to claim 50.
100. A microfluidic reactor comprising at least one microfluidic template and window plates attached to the template, the microfluidic template and window plates defining a plurality of reaction cells which provide for flow of liquid solution through the cells for parallel chemical reactions, each reaction cell comprising a first chamber in fluid communication with but spatially separated from a second chamber, the first chamber being adapted to be an illumination chamber, and the second chamber being adapted to be a reaction chamber for reaction of photo-generated products in the first chamber.
101. A microfluidic reactor according to claim 100, wherein the plates are attached by covalent attachment.
102. A microfluidic reactor according to claim 100, wherein the plates are attached by non-covalent attachment.
103. A microfluidic reactor according to claim 100, wherein the first and second chambers are in fluid communication by a connection channel.
104. A microfluidic reactor according to claim 100, wherein the first chamber is connected to an inlet channel, the second chamber connected to an outlet channel, and the plurality of reaction cells are connected by distribution channels for parallel chemical reactions.
105. A microfluidic reactor according to claim 104, wherein the first and second chambers are in fluid communication by a connection channel.
106. A microfluidic reactor according to claim 100, wherein the second chambers comprise at least one surface having immobilized molecules thereon.
107. A microfluidic reactor according to claim 100, wherein the second chambers comprise at least two surfaces having immobilized molecules thereon.
108. A microfluidic reactor according to claim 100, wherein the second chambers comprise a three dimensional array of surfaces having immobilized molecules thereon.
109. A microfluidic reactor according to claim 100, wherein the second chambers comprise immobilized oligonucleotides.
110. A microfluidic reactor comprising at least one microfluidic template and window plates attached to the template, the reactor providing at least one inlet channel, at least one outlet channel, and distribution channels, and a plurality of liquid flow-through photoillumination reaction cells for parallel chemical reactions.
111. A microfluidic reactor according to claim 110, wherein the plates are attached by covalent attachment.
112. A microfluidic reactor according to claim 110, wherein the plates are attached by non-covalent attachment.
113. A microfluidic reactor according to claim 110, wherein the reaction cells comprise at least one surface having immobilized molecules thereon.
114. A microfluidic reactor according to claim 110, wherein the reaction cells comprise at least two surfaces having immobilized molecules thereon.
115. A microfluidic reactor according to claim 110, wherein the reaction cells comprise a three dimensional array of surfaces having immobilized molecules thereon.
116. A microfluidic reactor according to claim 110, wherein the reaction cells comprise immobilized oligonucleotides.
117. A microfluidic reactor according to claim 110, wherein the reactor comprises a common inlet channel and a common outlet channel.
118. A microfluidic reactor according to claim 110, wherein the reactor comprises a common inlet channel.
119. A microfluidic reactor according to claim 110, wherein the reactor comprises a common outlet channel.
120. A microfluidic reactor comprising:
   a plurality of flow-through reaction cells in fluid communication with each other via
   distribution channels for parallel chemical reactions, each reaction cell comprising:
      i. at least one illumination chamber, and
      ii. at least one reaction chamber,
   wherein the illumination chamber and the reaction chamber are in flow communication and overlap with each other in the reaction cell.
121. A microfluidic reactor according to claim 120, wherein the overlap of chambers is a partial overlap.
122. A microfluidic reactor according to claim 120, wherein the overlap of chambers is a total overlap.
123. A microfluidic reactor according to claim 120, wherein the reaction cells are adapted for use of in situ generated chemical reagents.
124. A microfluidic reactor according to claim 120, wherein the reactor is adapted to avoid chemical intermixing between the reaction cells.
125. A microfluidic reactor according to claim 120, wherein the reactor comprises at least 10 reaction cells.
126. A microfluidic reactor according to claim 120, wherein the reactor comprises immobilized molecules.
127. A microfluidic reactor according to claim 126, wherein the immobilized molecules are selected from the group consisting of DNA, RNA, DNA oligonucleotides, RNA oligonucleotides, peptides, oligosaccharides, and phospholipids.
128. A microfluidic reactor according to claim 126, wherein the immobilized molecules are oligonucleotides.
129. A microfluidic reactor according to claim 120, wherein the reactor comprises a common inlet and a common outlet.
130. A high-density flowthrough multi-cell microfluidic reactor comprising a microfluidic template, at least one inlet channel, at least one outlet channel, and a plurality of flow through reaction cells for parallel chemical reactions, wherein the inlet channel and outlet channel are imbedded in the mid-section of the microfluidic template.
131. The reactor of claim 130, wherein each flow through reaction cell comprises a spatially separated illumination chamber and reaction chamber, which are in fluid communication with each other.
132. The reactor of claim 131, wherein the illumination chamber and reaction chamber are connected by a channel.
133. The reactor of claim 130, wherein the reaction chamber comprises immobilized molecules.
134. The reactor of claim 133, wherein the immobilized molecules are oligonucleotides.
135. A microfluidic reactor comprising a microfluidic template, a back plate attached to the template, and a window plate attached to the template, wherein the reactor comprises a plurality of flow-through reaction cells in fluid communication with an inlet channel and an outlet channel for parallel chemical reactions, wherein the inlet channel and the outlet channel are located between the back plate and the microfluidic template.
136. The reactor according to claim 135, wherein the reactor further comprises a shadow mask on the window plate.
137. The reactor according to claim 135, wherein the reaction cells comprise immobilized molecules.
138. The reactor according to claim 137, wherein the immobilized molecules are oligonucleotides.
139. The reactor according to claim 137, wherein the immobilized molecules are disposed on at least two surfaces of the reaction cell.
140. A microfluidic reactor comprising a plurality of flow-through photoillumination reaction cells for parallel chemical reactions in fluid communication with at least one inlet channel and at least one outlet channel, wherein the reaction cells are connected to fluid distribution channels in parallel which comprise a through-hole at their end so that fluid can flow through the channel without passing through the reaction cells.
141. A microfluidic reactor according to claim 140, wherein the through hole is in fluid communication with the outlet channel.
142. A microfluidic reactor according to claim 140, wherein the reaction cells comprise a photoillumination chamber and a reaction chamber which are in fluid communication and are spatially separated.
143. A microfluidic reactor according to claim 140, wherein the reaction cells comprise a photoillumination chamber and a reaction chamber which partially overlap with each other.
144. A microfluidic reactor according to claim 140, wherein the reaction cells comprise a photoillumination chamber and a reaction chamber which completely overlap with each other.
145. A microfluidic reactor comprising a plurality of flow-through photoillumination reaction cells for parallel chemical reactions in fluid communication with at least one inlet channel and at least one outlet channel, wherein the reaction cells are connected in parallel with fluid distribution channels, wherein each reaction cell has a separate outlet channel which allows for individual collection of effluent from each reaction cell.
146. A microfluidic reactor according to claim 145, wherein the reaction cells comprise a photoillumination chamber and a reaction chamber which are in fluid communication and are spatially separated.
147. A microfluidic reactor according to claim 146, wherein the photoillumination chamber and the reaction chamber are connected by a connection channel.
148. A microfluidic reactor according to claim 145, wherein the reaction cells comprise a photoillumination chamber and a reaction chamber which partially overlap with each other.
149. A microfluidic reactor according to claim 145, wherein the reaction cells comprise a photoillumination chamber and a reaction chamber which completely overlap with each other.
150. A microfluidic reactor adapted for in situ use of photogenerated reagents, wherein the reactor comprises an inlet channel , an illumination chamber, a connection channel, a reaction chamber, and an outlet channel, wherein the illumination chamber connects with the inlet channel, the connection channel connects the illumination chamber and the reaction chamber, and the outlet channel connects with the reaction chamber.
151. Use of the reactor according to claim 1 in making chemical compounds.
152. Use of the reactor according to claim 50 in making chemical compounds.
153. Use of the reactor according to claim 1 in screening chemical compounds.
154. Use of the reactor according to claim 50 in screening chemical compounds.
155. Use of the reactor according to claim 1 in assaying chemical compounds.
156. Use of the reactor according to claim 50 in assaying chemical compounds.
157. A method of making the reactor according to claim 1 comprising the step of photolithographically producing a microfluidic template which is adapted for bonding to one or more windows.
158. A method of making the reactor according to claim 50 comprising the step of photolithographically producing a microfluidic template which is adapted for bonding to one or more windows.
159. A method for enhancing parallel photochemical reactivity in a microfluidic reactor having a plurality of isolated reaction cells, said method comprising the step of providing spatially separated or overlapping illumination and reaction chambers in each reaction cell.

## Claims

1. A microfluidic reactor comprising:
a plurality of flow-through reaction cells for parallel chemical reactions, each reaction cell comprising:
i. at least one illumination chamber, and
ii. at least one reaction chamber,
wherein the illumination chamber and the reaction chamber are in flow communication
and are spatially separated in the reaction cell; and
wherein said at least one illumination chamber receives a photogenerated acid precursor and is illuminated to produce a photogenerated acid.

2. A microfluidic reactor according to claim 1, wherein the reactor comprises at least 10 reaction cells, at least 100 reaction cells, at least 1,000 reaction cells, at least 10,000 reaction cells, or 900 to 10,000 reaction cells.

3. A microfluidic reactor according to claim 1, wherein the reactor comprises a silicon or plastic microfluidic template.

4. A microfluidic reactor according to claim 1, wherein a distance between reaction cells which are adjacent to each other is 10 to 5,000 microns, 10 to 2,000 microns, 10 to 500 microns, 10 to 200 microns, or larger than 5,000 microns.

5. A microfluidic reactor according to claim 1, wherein the reactor comprises a microfluidic template and at least one window plate.

6. A microfluidic reactor according to claim 1, wherein the reactor further comprises at least one shadow mask.

7. A microfluidic reactor according to claim 1, wherein the reactor further comprises an inlet channel and an inlet restriction gap connected to the illumination chamber, and an outlet channel and an outlet restriction gap connected to the illumination chamber.

8. A microfluidic reactor according to claim 1, wherein the reactor further comprises inlet channels and inlet restriction gaps in fluid communication with the illumination chambers of the reaction cells, and wherein the reactor further comprises outlet channels and outlet restriction gaps in fluid communication with the reaction chambers of the reaction cells, and wherein illumination chambers and reaction chambers of the reaction cells are connected by connection channels.

9. A microfluidic reactor according to claim 1, wherein the reactor further comprises one common inlet channel, branch inlet channels, branch outlet channels, and one common outlet channel.

10. A microfluidic reactor according to claim 1, wherein the reactor further comprises immobilized molecules in the reaction chamber.

11. A microfluidic reactor according to claim 21, wherein the immobilized molecules are oligonucleotides.

12. A microfluidic reactor according to claim 1, wherein the reaction chambers are in capillary form.

13. A microfluidic reactor according to claim 31, wherein the reaction chambers in capillary form have diameters of 0.05 micrometers to 500 micrometers or 0.1 micrometers to 100 micrometers.

14. A microfluidic reactor according to claim 1, wherein the reactor comprises an array of oligonucleotides in the reaction chamber, a microfluidic template made of silicon, and first and second window plates made of glass and attached to the template.

15. A microfluidic reactor according to claim 1, wherein the device comprises an array of oligonucleotides in the reaction chambers, a microfluidic template made of silicon, window plates, a shadow mask, inlet channels and inlet restriction gaps connected to the illumination chambers, outlet channels and outlet restriction gaps connected to the reaction chambers, distribution channels for parallel reactions in the reaction cells, and connection channels to connect illumination and reaction chambers.
